# EUROPEAN PATENT APPLICATION

(11) **EP 4 563 703 A1**
(43) Date of publication of application: **04.06.2025**
(21) Application number: 23846282.4
(22) Date of filing: 14.07.2023
(51) Int. Cl.: C12N 15/62, A61K 35/15, A61K 35/17, A61P 35/00, C12N 5/0783, C12N 5/10, C12N 15/12, C12N 15/13

(54) **ARTIFICIAL RECEPTOR HAVING SHEDDING STRUCTURE**

(30) Priority: 26.07.2022 JP 2022118674
(71) Applicant: Kyoto University, Kyoto-shi, Kyoto 606-8501 (JP)
(72) Inventor: KANEKO, Shin, Kyoto-shi, Kyoto 606-8501 (JP); MINAGAWA, Atsutaka, Kyoto-shi, Kyoto 606-8501 (JP)
(74) Representative: J A Kemp LLP
(86) International application number: PCT/JP2023/026123
(87) International publication number: WO 2024/024551

(57) **Abstract**

The present invention provides: an artificial receptor including a ligand-binding site, a transmembrane domain having a shedding structure, and a signal transduction domain into an immune cell; and a nucleic acid encoding the artificial receptor.

## Description

### TECHNICAL FIELD

The present invention relates to an artificial receptor having a shedding structure. More specifically, the present invention relates to an artificial receptor that includes a ligand-binding site, a transmembrane domain having a shedding structure, and a signal transduction into an immune cell. Also, the present invention relates to a method for designing a receptor with regulated trogocytosis effect or a nucleic acid encoding the receptor.

### BACKGROUND ART

A phenomenon is widely known in which continuous input of strong stimuli leads to a decrease in sensitivity. It is considered that such a decrease in sensitivity of a receptor caused by continuous signaling has biological significance, and shedding is known as one of mechanisms by which the decrease in sensitivity is expected to be prevented. The shedding is a mechanism in which the extracellular domain of a receptor is cleaved by a metalloprotease. It is known that, even in only tyrosine kinase receptors that have been ever tested, half or more of them undergo the shedding (Non Patent Literature 1). It is considered that, after a reaction of a receptor with a ligand causes phosphorylation of an intracellular domain, the reaction is once reset through the shedding and thus signals are adjusted, but details of how the signals are controlled through the shedding remain unknown.

A T cell receptor (TCR) that is used in cancer immunotherapy and through which a T cell recognizes a target is also one type of the tyrosine kinase receptors. As one of signal regulate mechanisms, it is known that the cell-surface expression level of a TCR-CD3 complex decreases when a stimulus is applied to the TCR. An artificial chimeric antigen receptor (CAR) that is known to have revolutionary therapeutic effects in immune cell therapy has a structure with which an antigen is recognized through binding of an antibody to a coreceptor such as a CD3 intracellular domain, a CD28, or a 4-1BB and thus a TCR signal is transmitted to the T cell, and all of the CD3 and costimulatory molecules are tyrosine kinase-type receptors.

It is known that continuously conducting CAR cell therapy leads to reduced therapeutic effects. Trogocytosis is known as one of mechanisms that lead to reduced effects of the CAR cell therapy and further a recurrence after the treatment (Non Patent Literature 2). The trogocytosis is a mechanism in which a reaction between a target cell and a CAR-expressing T cell causes a membrane exchange by nibbling the membrane of the target cell at the reaction sites and thus the T cell gains the target antigen. Accordingly, in the CAR cell therapy, antigen expression in cancer cells decreases and thus immune escape occurs, which causes recurrence. In addition, it is also reported that the T cell that gained the cancer antigen expresses the gained cancer antigen on its cell membrane and is thus targeted by other CAR-T cells, which leads to suppression of an immune reaction. The trogocytosis was discovered as a membrane exchange phenomenon induced by an immune reaction, and is known to be a physiological phenomenon conserved from protista such as ameba cells onward. However, details of the membrane exchange mechanism remain unknown, and a means to suppress the trogocytosis in the CAR-T cell therapy and solve the problem has not been known yet.

### Citation List

### Non Patent Literature

Non Patent Literature 1: Merilahti JAM and Elenius K, Oncogene 38(2): 151-163 (2019)
Non Patent Literature 2: Hamieh M, et al., Nature. 568(7750): 112-116 (2019)

### SUMMARY OF INVENTION

### Technical Problem

Therefore, it is an object of the present invention to provide a method for suppressing trogocytosis between cells in order to establish immunotherapy for diseases such as cancers that is likely to prevent a recurrence and to retain therapeutic effects.

### Solution to Problem

In order to solve the aforementioned problems, the inventors of the present invention focused on CAR signal control mechanisms. First, the inventors of the present invention investigated how the immune cell therapy was affected when the structure of the transmembrane domain of a CAR was changed to a structure capable of undergoing the shedding (also referred to as a "shedding structure") to bring the structure of the CAR close to that of a physiological tyrosine kinase receptor. It was also considered that the CAR having the shedding structure lacked a site recognizing an antigen presented on the surface of the cancer cell due to the shedding and thus had a reduced ability to recognize a cancer cell. Actually, it was shown that, when a CAR-expressing cell and a cancer cell were reacted with each other for a short period of time, 3 hours, the reactivity of the CAR having the shedding structure decreased, even to a small extent, compared with a CAR that did not have the shedding structure. However, it was surprisingly demonstrated that, when a CAR-expressing cell and a cancer cell were reacted with each other for a long period of time, the reactivity of the CAR having the shedding structure was enhanced compared with a normal CAR, and antitumor effects were also enhanced in a tumor-transplanted mouse used as an in-vivo model. When further investigation was conducted based on these findings, it was revealed that the CAR having the shedding structure prevented trogocytosis of a target antigen in a cancer cell through the shedding. It was found that one of the reasons why the antitumor effects were enhanced in the CAR having the shedding structure was that a long-term reactivity was maintained due to suppression of trogocytosis.

Furthermore, the inventors of the present invention also focused on the intracellular domain of the CAR, and hypothesized that the trogocytosis could be further regulated by adjusting the size of the domain, and the signal transduction domain was not necessarily required to regulate the trogocytosis. As a result of conducting a study based on these hypotheses, it was demonstrated that the signal transduction domain was not essential for the occurrence of the trogocytosis, the intensity of the trogocytosis varied depending on the size of the intracellular domain, and the signal transduction domain more strongly induced the trogocytosis than a non-signal transduction domain when these domains had the same size. It was also demonstrated that the intensity of the trogocytosis could be further regulated by combining the application of the shedding structure and changes in the number and the order of intracellular signal transduction domains. It can be said that a technique for adjusting the trogocytosis level of a CAR was successfully established based on these findings. As a result of further conducting a study based on these findings, the inventors of the present invention achieved the present invention.

That is to say, the present invention is as follows.
[1] An artificial receptor, comprising a ligand-binding site, a transmembrane domain having a shedding structure, and a signal transduction domain into an immune cell.
[2] The artificial receptor according to [1], which is selected from the group consisting of a chimeric antigen receptor, a modified T cell receptor, a modified Fc receptor, and a modified tyrosine kinase receptor.
[3] The artificial receptor according to [1] or [2], wherein the immune cell is selected from the group consisting of a T cell, a natural killer cell, and a macrophage.
[4] The artificial receptor according to [3], wherein the T cell is a cytotoxic T cell.
[5] The artificial receptor according to any one of [1] to [4], wherein the shedding structure is derived from a protein selected from the group consisting of a notch protein, an amyloid precursor protein, and CD28.
[6-1] The artificial receptor according to any one of [1] to [5], wherein the signal transduction domain into an immune cell is derived from at least one type selected from the group consisting of a CD3ζ chain, CD28, and 4-1BB.
[6-2] The artificial receptor according to any one of [1] to [6-1], wherein the ligand-binding site is a cancer antigen-binding site.
[7-1] The artificial receptor according to any one of [1] to [6-2], wherein the transmembrane domain having a shedding structure comprises
   (1) an amino acid sequence represented by SEQ ID NO: 26, or
   (2) an amino acid sequence obtained by substituting, deleting, inserting, and/or adding one or several amino acids in the amino acid sequence of (1).
[7-2] The artificial receptor according to any one of [1] to [7-1], comprising an extracellular hinge domain having an amino acid sequence consisting of 29 or less amino acids.
[8-1] The artificial receptor according to any one of [1] to [7-2], having no γ-secretase cleavage site in the transmembrane domain.
[8-2] The artificial receptor according to any one of [1] to [8-1], having no γ-secretase cleavage site in an intracellular domain.
[9-1] The artificial receptor according to any one of [1] to [8-2], comprising two or more identical signal transduction domains.
[9-2] The artificial receptor according to [9-1], wherein the signal transduction domain is a signal transduction domain of CD28.
[10-1] A nucleic acid encoding the artificial receptor according to any one of [1] to [9-2].
[10-2] A vector comprising the nucleic acid according to [10-1].
[11-1] An immune cell comprising the artificial receptors according to any one of [1] to [9-2], the nucleic acid according to [10-1], or the vector according to [10-2].
[11-2] The immune cell according to [11-1], which is selected from the group consisting of a T cell, a natural killer cell, and a macrophage.
[12-1] A medicine comprising the immune cell according to [11-1] or [11-2].
[12-2] The medicine according to [12-1] for treatment or prevention of a cancer.
[13] A method for designing a receptor with regulated trogocytosis effect or a nucleic acid encoding the receptor, comprising
   (1) a step of selecting a receptor comprising a ligand-binding site, a transmembrane domain, and a signal transduction domain into an immune cell, and
   (2A) a step of adjusting sensitivity of the transmembrane domain selected in the step (1) to shedding, and/or
   (2B) a step of adding, inserting, and/or deleting one or more amino acid residues in the signal transduction domain of the receptor prepared in the step (1), and/or substituting one or more amino acid residues in the signal transduction domain.
[14] A method for producing a receptor with regulated trogocytosis effect or a nucleic acid encoding the receptor, comprising
   (1) a step of preparing a receptor that includes a ligand-binding site, a transmembrane domain, and a signal transduction domain into an immune cell, and
   (2A) a step of adjusting sensitivity of the transmembrane domain prepared in the step (1) to shedding, and/or
   (2B) a step of adding, inserting, and/or deleting one or more amino acid residues in the signal transduction domain of the receptor prepared in the step (1), and/or substituting one or more amino acid residues in the signal transduction domain.
[15-1] The method according to [13] or [14], wherein the receptor selected or prepared in the step (1) is selected from the group consisting of a chimeric antigen receptor, a T cell receptor, an Fc receptor, and a tyrosine kinase receptor.
[15-2] The method according to any one of [13] to [15-1], wherein the immune cell is selected from the group consisting of a T cell, a natural killer cell, and a macrophage.
[16] A method for treating or preventing a cancer in a mammal, comprising administering an effective amount of the immune cell according to [11-1] or [11-2] or the medicine according to [12-1] or [12-2] to the mammal.
[17] The immune cell according to [11-1] or [11-2] or the medicine according to [12-1] or [12-2] for use in the treatment or prevention of a cancer.
[18] Use of the immune cell according to [11-1] or [11-2] or the medicine according to [12-1] or [12-2] in the manufacture of a therapeutic or preventive medicine for a cancer.

### Advantageous Effects of Invention

Using the present invention makes it possible to prevent trogocytosis in immune cells, allow a receptor such as a CAR or a TCR to maintain the reactivity for a long period of time, and greatly enhance the effects of the existing T cell therapy. Also, modifying the transmembrane domain and the intracellular domain of a receptor makes it possible to regulate trogocytosis. Furthermore, the findings of the present invention can be useful for elucidating parts of the mechanism of universal physiological phenomena, such as the role of trogocytosis in a living organism and the reason why a tyrosine kinase receptor has a structure capable of undergoing shedding.

### Brief Description of Drawings

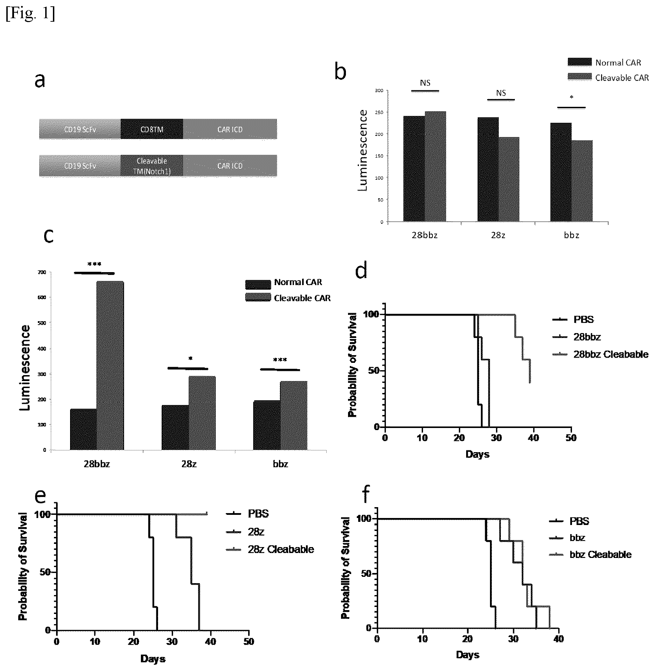

[FIG. 1] A CAR (Cleavable-CAR) having a shedding structure was likely to maintain the reactivity for a long period of time and was also more effective in vivo. a: Structures of Cleavable-CAR construct and control (Normal-CAR) construct. The transmembrane domain (TM) of a Notch1 protein was used as the shedding structure (also referred to as a "cleavable structure"), and a CAR (Normal-CAR) having a common CD8 TM was used as the control. b: Various CAR constructs were introduced into NFAT-Luc jurkat (Promega), and the resulting cells were cocultured with 4×10⁵ NALM6 cells at a ratio of 1:1 for 3 hours (3 h). Then, luciferin was added, and the luminescence intensity was measured. After 3 h, 28bbz (a CAR constructed by coupling an ScFv for CD19 (i.e., CD19-recognizing ScFv) (CD19 ScFv), the signal transduction domain of CD28, the signal transduction domain of 4-1BB, and the signal transduction domain of the CD3ζ chain) and 28z (a CAR constructed by coupling the CD19 ScFv, the signal transduction domain of CD28, and the signal transduction domain of the CD3ζ chain) exhibited nearly equal reactivity, and it was observed that the reactivity of bbz (a CAR constructed by coupling the CD19 ScFv, the signal transduction domain of 4-1BB, and the signal transduction domain of the CD3ζ chain) decreased to a small extent. c: Results of measurements of luminescence intensity after coculture was continued for 4 days in the same experimental system as in b. It was confirmed that, after the long-term culture, Cleavable-CARs had higher reactivity than Normal CARs irrespective of the type of intracellular domain (ICD). d, e, f: Survival curve after 5×10⁵ NALM6 cells were intravenously transplanted to 6- to 12-week-old NSG mice on Day 0 and then 2×10⁵ various CAR-introduced peripheral blood CD8 cells were intravenously administered to the mice on Days 4 and 10. d shows the comparison of 28bbz and 28bbz-Cleavable, e shows the comparison of 28z and 28z-Cleavable, and f shows the comparison of bbz and bbz-Cleavable. No significant difference was observed in the case of bbz, and it was observed that Cleavable-CAR caused obviously significant life extension in the cases of 28z and 28bbz.

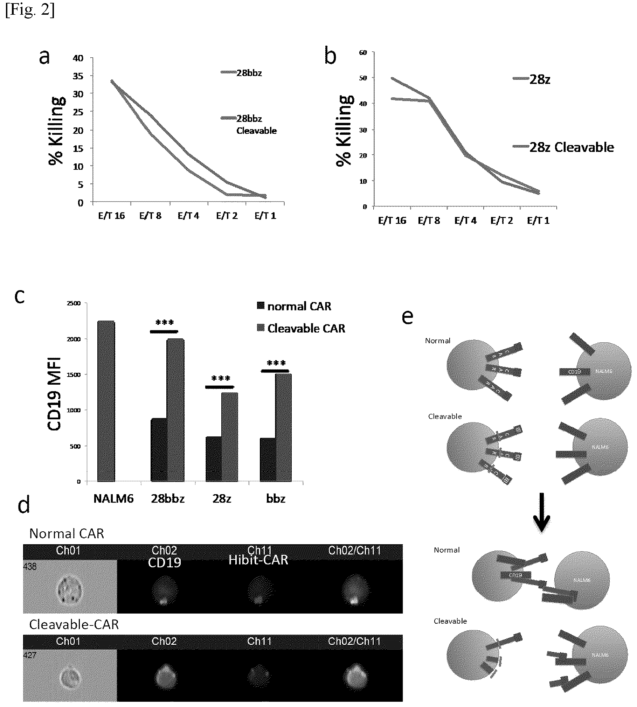

[FIG. 2] Cleavable-CAR suppresses trogocytosis. a, b: Cytotoxicity assay of NALM6 using various CAR-introduced PBMC. CAR-T and NALM6 were cocultured at various Effector/Target ratios and were analyzed in accordance with the protocol of the cytotoxicity assay kit (TECHNO SUZUTA Co., Ltd.). No obvious differences were observed. c: NALM6 cells and CAR-introduced jurkat cells were cocultured at a ratio of 1:1 overnight, and the expression level of CD19 in the NALM6 was analyzed through flow cytometry. It was demonstrated that a decrease in CD19 (trogocytosis) was significantly suppressed in the case of Cleavable-CAR compared with the case of Normal-CAR. d, e: Photographs (d) and schematic diagrams (e) of NALM6 after Hibit-tags (Promega) were added to the termini of CARs in jurkat cells into which 28bbz Normal-CARs or 28bbz Cleavable-CARs had been introduced, the jurkat cells were cocultured with NALM6 and fixed, and then the cells stained with Mouse Anti-Hibit mAb (Promega), AlexaFluor647 goat anti-mouse igG (Invitrogen), and a CD19-FITC antibody were subjected to imaging analyses using image-Stream (MKII). It was observed that CD19-FITC decreased and CARs were taken into the cells in the case of Normal-CAR, whereas it was confirmed that CD19 did not decrease and CARs were present on the surface of the cell membrane in the case of Cleavable-CAR.

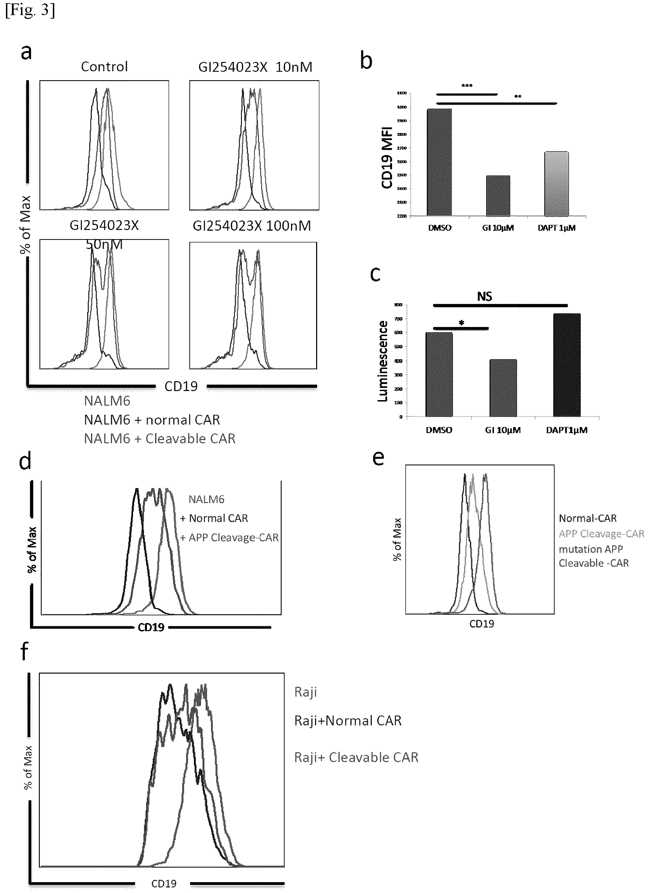

[FIG. 3] Cleavage of extracellular domain is important for suppression of trogocytosis and maintenance of long-term signals. a: Results of FACS analysis of CD19 expression in NALM6 after Jurkat cells expressing Cleavable-CARs (28bbz) or Normal-CARs and NALM6 cells were cocultured. When an ADAM inhibitor GI254023X, which induces the cleavage of the extracellular domain, was added at various concentrations, it was confirmed that there was no change in the case of Normal-CAR, whereas the CD19 expression decreased in a concentration-dependent manner in the case of coculture with Cleavable-CAR. It was demonstrated that the ADAM inhibition could cause trogocytosis even in the case of Cleavable-CAR. b: Trogocytosis level when an ADAM inhibitor or a γ-secretase inhibitor was added to the medium in which Jurkat expressing Cleavable-CAR (28bbz) and NALM6 were cocultured. Trogocytosis was easily caused by both the ADAM inhibition and the γ-secretase inhibition, but was more easily caused by the ADAM inhibition. c: Results of luminescence detection after NFAT jurkat cells expressing Cleavable-CAR (28bbz) and NALM6 were cocultured at a ratio of 1:1 in the presence of various inhibitors for 4 days, and then luciferin was added thereto. A decrease in the signal was observed in the case of the ADAM inhibitor, whereas a decrease was not observed in the case of the γ-secretase inhibitor and rather the signal could be enhanced though there was no significant difference. d: Trogocytosis in Cleavable-CAR with TM of amyloid precursor protein (APP). It was demonstrated that the shedding structure was not limited to Notch and trogocytosis could be suppressed using any shedding structure. e: It was shown that trogocytosis was further suppressed by using an APP structure into which a mutation (Swe mutation (APP695, K595N/M596L APP double mutant)) that enhances shedding of the extracellular domain. It was confirmed that the degree of shedding controlled the trogocytosis level. f: Results of analysis of CD19 expression in Raji cells, a CD19-expressing cell line, cocultured with Normal or Cleavable 28bbz CAR. It was confirmed that trogocytosis was also suppressed in Raji cells in the case of Cleavable-CAR.

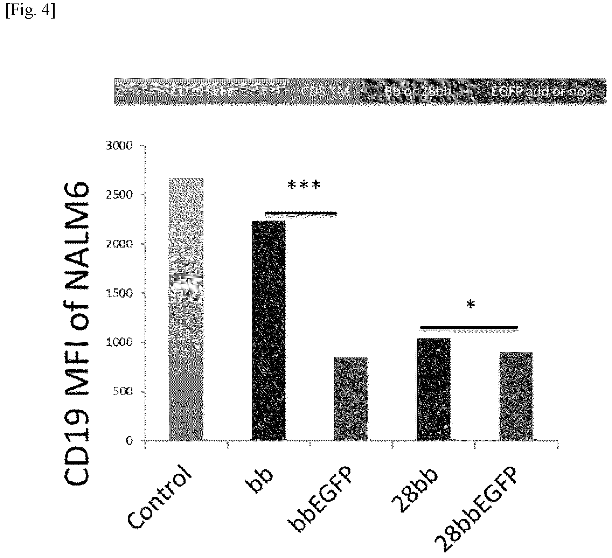

[FIG. 4] Trogocytosis level can vary depending on the size of the intracellular domain of a receptor. In order to investigate what factor is important for the degree of trogocytosis, first, jurkat expressing a CAR with no CD3ζ (only bb (a CAR constructed by coupling the CD19 ScFv and the signal transduction domain of 4-1BB) or 28bb (a CAR constructed by coupling the CD19 ScFv, the signal transduction domain of CD28, and the signal transduction domain of 4-1BB)) and NALM6 were cocultured, and then the CD19 expression in the NALM6 was analyzed. Surprisingly, trogocytosis was observed even when no CD3ζ was included. 28bb induced trogocytosis more strongly than 28. The inventors thought that the length or weight of the intracellular domain was important for trogocytosis rather than an increase in signals, and produced bbGFP and 28bbGFP having the C-terminus with GFP and subjected them to coculture in the same manner as above. As a result, it was confirmed that they significantly increased trogocytosis compared with bb and 28bb.

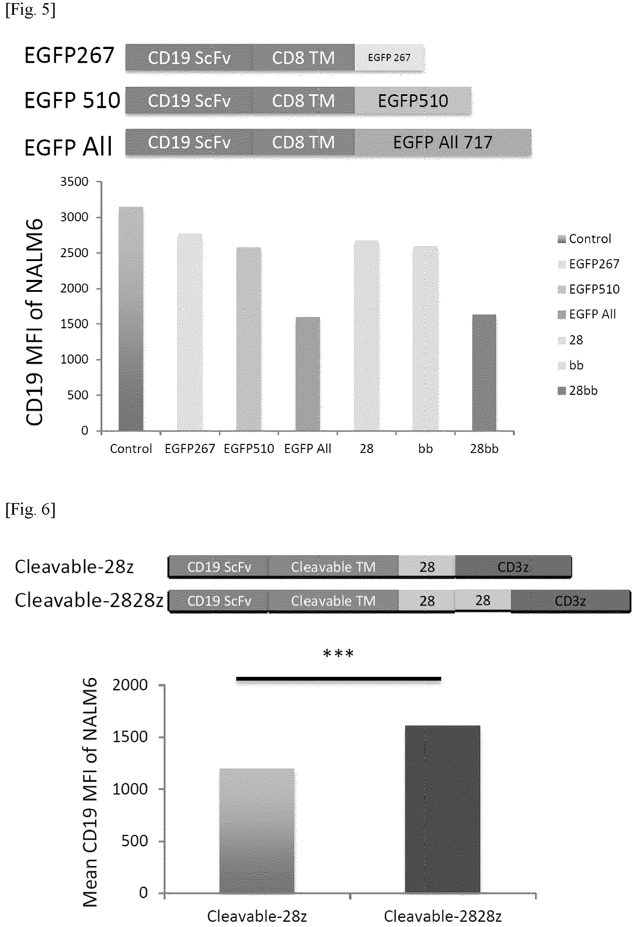

[FIG. 5] CARs having an intracellular domain to which a portion of EGFP protein had been added downstream of CD19ScFv-CD8TM were produced and cocultured with NALM6, followed by measurement of the average value of the CD19 expression levels in the NALM6. Since it is obvious that EGFP has no signal transduction function, it was shown that trogocytosis occurred without a signal transduction domain. Compared with the case where a portion of EGFP, about 1/3 of the entire length (EGFP267) or about 2/3 of the entire length (EGFP510), was added, trogocytosis was enhanced in the length-dependent manner, and it was thus demonstrated that trogocytosis was likely to occur in the intracellular domain size-dependent manner. Furthermore, when the non-signal transduction domain (EGFP267) and the tyrosine kinase domain (28bb), which have the same length, were compared, it was found that trogocytosis was more strongly induced in the case of the tyrosine kinase domain, which suggests that, among domains having the same length, signal transduction domains have enhanced traction.
[FIG. 6] It was possible to more strongly suppress trogocytosis by lining up signal domains having the same size near a portion of the membrane including the cleavage site as in 2828 (a domain formed by linking the CD28 signal transduction domains in tandem). It is thought that the reason why trogocytosis was suppressed more strongly is that the traction during signal transduction increased. It was found that the trogocytosis level can be controlled by changing the arrangement or number of intracellular domains in a CAR having the shedding structure in such a manner.

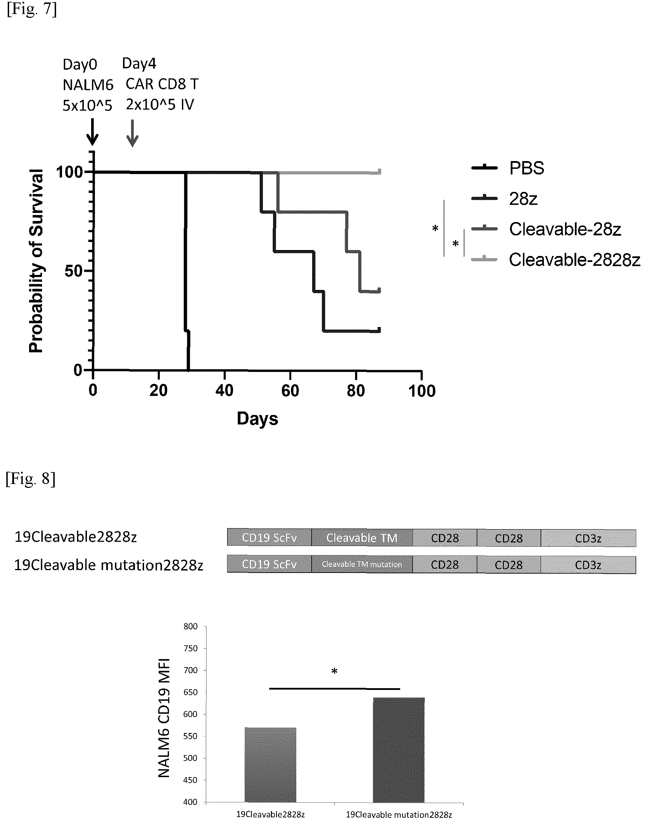

[FIG. 7] It was observed that the antitumor activity was significantly enhanced in vivo by linking CD28 intracellular signal domains in tandem in the Cleavable sequence derived from mouse notch1 (Cleavable-28bbz), compared with the case where a single CD28 intracellular signal domain was included (Cleavable-2828z).
[FIG. 8] Results of CD19 expression in NALM6 after coculture of NALM6 cells and CAR-expressing Jurkat cells. As a result of comparing 19Cleavable2828z (Cleavable-2828z having the CD19 ScFv) and 19Cleavable mutation 2828z (19Cleavable2828z configured not to be cleaved by γ-secretase by introducing deletion mutation into 19Cleavable2828z (deleting the region in SEQ ID NO: 28 from amino acid residue 329 to amino acid residue 335)), trogocytosis was further suppressed when the mutant was used. It is thus considered that the trogocytosis suppressing effects are obtained through the cleavage of the extracellular domain.

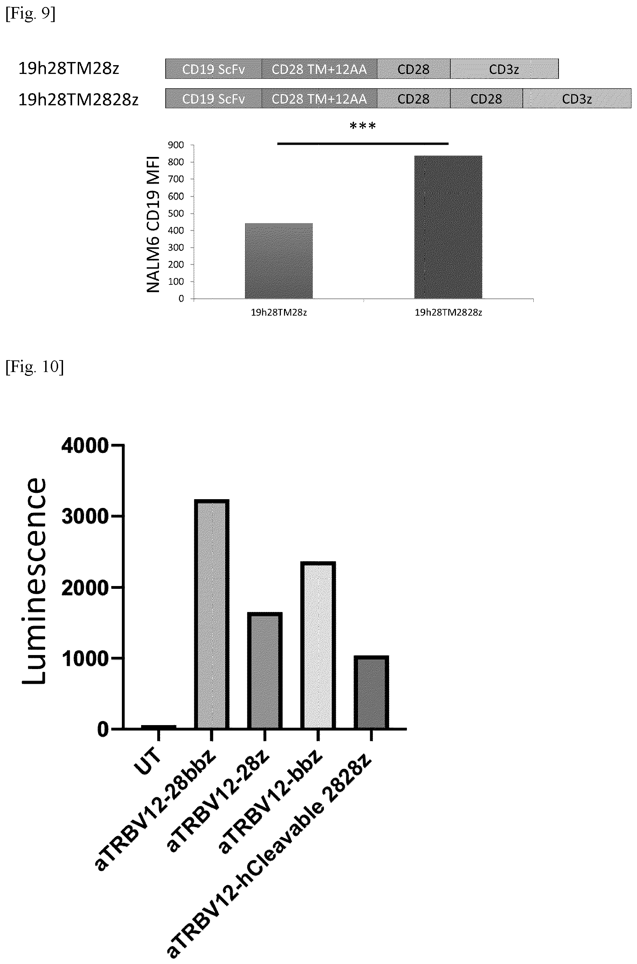

[FIG. 9] It was found that, in the same trogocytosis detection system, trogocytosis was also further suppressed when signal domains were linked in tandem in a CAR in which the human CD28 TM and 12 amino acids on the upstream side thereof (included in a region from amino acid residue 141 to amino acid residue 152 of CD28) were used as the TM site. Accordingly, it is expected that CD28 is also a shedding protein.
[FIG. 10] Jurkat cells express a T cell receptor TRBV12. NFAT-Jurkat cells and various CAR-T cells transduced with an ScFv for TRBV12 (aTRBV12 ScFv) were cocultured, and then the TCR signal intensity on the NFAT-jurkat was detected. It was confirmed that the signals tended to decrease when hCleavable-2828z CAR was used as the CAR. It is suggested that making the sequence of a receptor a cleavable sequence can lead to escape from immune cells and the like. "UT" refers to a control cocultured with cells expressing no CARs.

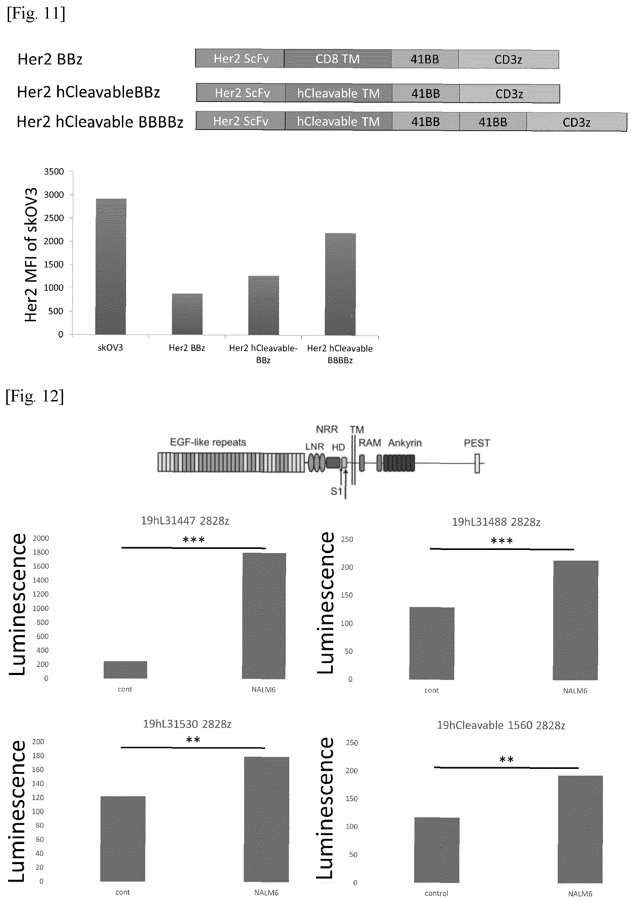

[FIG. 11] Measurement results of a tandem effect of Cleavable-CAR and a signal domain in ScFV for Her2 (Her2 ScFV). The Her2 expression on the Her2-expressing tumor line skOV3 after overnight coculture of the skOV3 and various Her2-CARs was analyzed through FACS. It was shown that, when hCleavable was used, an increase in the number of signal domains in the Her2-CAR also led to the suppression of trogocytosis. "skOV3" on the left side of the graph indicates the control Her2 expression level after coculture with cells into which CARs were not introduced.
[FIG. 12] Test results regarding Human Notch1 protein length. The structure of Notch1 is shown on the upper side of the diagram. CARs (19hL31447 2828z, 19hL31448 2828z, 19hL31530 2828z, and 19hL31560 2828z) that respectively have the amino acid residues at position 1447, position 1488, position 1530, and position 1560 of human Notch1 (NCBI Accession No.: NP_060087.3) as the first amino acid residue of the transmembrane domain having the shedding structure were produced and introduced into NFAT-Jurkat, and a control or NALM6 was cocultured with the obtained NFAT-Jurkat. After the coculture, the reactivity was checked based on the comparison of the NFAT-Jurkat. The reactivity of the CAR having all the LNR, which has the amino acid residue at position 1447 as the first amino acid of the transmembrane domain, was as strong as that of hCleavable. As the LNR was shortened, the reactivity became weaker, but a significant reaction was observed compared with the control. Accordingly, it is considered that changing the length of the Notch1 sequence used makes it possible to control the reactivity. "Control (cont)" indicates the luminescence intensity when coculture was not conducted.

### DESCRIPTION OF EMBODIMENTS

### 1. Artificial Receptor with Transmembrane Domain Having Shedding Structure

The present invention provides an artificial receptor with a transmembrane domain (also called "transmembrane region" or "transmembrane site") having a shedding structure. More specifically, the present invention provides an artificial receptor (which may be referred to as "receptor of the present invention" hereinafter) that comprises a ligand-binding site, a transmembrane domain having a shedding structure, and a signal transduction domain into an immune cell. Typically, the receptor of the present invention comprises an extracellular hinge domain that links the ligand-binding site and the transmembrane domain. Due to the receptor of the present invention having the shedding structure, occurrence of trogocytosis between a cell with the receptor and a cell expressing a ligand recognized by the receptor can be suppressed, compared with a receptor that does not have this structure. The trogocytosis is a phenomenon that mainly occurs in immune cells and in which one cell nibbles off a protein on another cell together with a membrane.

In this specification, the term "receptor" means a structure that comprises a ligand-binding site, a transmembrane domain, and an intracellular domain, and the term "artificial receptor" means a receptor other than a natural receptor, that is to say, a receptor that is not present in a living mammal unless it is exogenously introduced. Specific examples of the receptor include a chimeric antigen receptor, a T cell receptor, an Fc receptor, and a tyrosine kinase receptor, but there is no limitation thereto. Some receptors (e.g., CAR and the like) are functional (have at least a ligand-binding ability) in the form of a monomer in vivo, and other receptors (e.g., TCR and the like) are functional in the form of a polymer composed of two or more monomers. The receptor described in this specification may be a constituent element of a receptor that is functional in the form of a polymer, but typically means a form that is functional in a living body. For example, the TCR receptor typically means a dimer composed of an α chain and a β chain or a dimer composed of a γ chain and a δ chain, or a complex composed of the dimer and CD3 bound thereto.

The ligand-binding site of the receptor is not particularly limited as long as it forms a portion of the extracellular domain and has an ability to bind to a ligand, and examples thereof include an antigen-binding site of an antibody, a single-chain variable fragment (scFv) formed by linking a heavy chain variable region (VH) and a light chain variable region (VL), which are included in the antigen-binding site, via a linker peptide, an antigen-binding site of a T cell receptor (TCR), a ligand-binding site of a tyrosine kinase receptor other than the TCR, and an immunoglobulin Fc-binding site of an Fc receptor. It is preferable that the ligand binding site does not have an ability to bind to, for example, the artificial receptor or a cell expressing the artificial receptor. That is to say, it is preferable that the artificial receptor does not include a ligand for the ligand-binding site thereinside. The artificial receptor may have one or more ligand-binding sites.

The ligand is typically a cell surface antigen of a cancer cell, but is not limited thereto. Examples of the ligand include BCMA, B7-H3, B7-H6, CD7, CD10, CD19, CD20, CD22, CD23, CD24, CD30, CD33, CD34, CD38, CD41, CD44, CD56, CD70, CD74, CD97, CD123, CD133, CD138, CD171, CD248, CAIX, CEA, c-Met, CS1 (CD319), CSPG4, CLDN6, CLD18A2, CYP1B1, DNAM-1, GD2, GD3, GM2, GFRα4, GPC3, GPR20, GPRC5D, globoH, Gp100, GPR20, GPRC5D, EGFR, EGFR variants, EpCAM, EGP2, EGP40, FAP, FITC, HER2, HER3, HPV E6, HPV E7, hTERT, IgG κ chains, IL-11Ra, IL-13Ra2, KIT, Lewis A, Lewis Y, Legumain, LMP1, LMP2, Ly6k, LICAM, MAD-CT-1, MAD-CT-2, MAGE-A1, MUC1, MUC16, NA-17, NY-BR-1, NY-ESO-1, O-acetyl-GD2, h5T4, PANX3, PDGRFb, PLAC1, polysialic acid, PSCA, PSMA, RAGE1, ROR1, sLe, SSEA-4, TARP, TAG-72, TEM7R, Tn antigens, TRAIL receptors, TRP2, TSHR, α fetoprotein, mesothelin, folate receptor α (FRα), folate receptor β (FRβ), FBP, UPK2, VEGF-R2, WT-1, and TCR (e.g., TRBV12 and the like).

The transmembrane domain of the receptor means a protein domain that penetrates the cell membrane. In this specification, the transmembrane domain refers to a region that penetrates the cell membrane, but the transmembrane domain may also include a portion of the extracellular domain or a portion of the intracellular domain. The transmembrane domain typically has an α-helix topology structure, but may also be a domain having a β-barrel-type structure or another structure. The term "transmembrane domain having a shedding structure" means a transmembrane domain having, inside the extracellular domain region (in other words, a portion of the extracellular domain near the membrane) or the transmembrane domain, a region cleavable by a cell-intrinsic sheddase. The transmembrane domain having a shedding structure typically includes at least a portion of an extracellular hinge domain and a transmembrane domain. The sheddase is a membrane-associated protein having an ability to cleave a portion of a transmembrane protein having a shedding structure. When the receptor of the present invention includes an extracellular hinge domain (which may also be referred to merely as a "hinge domain" hereinafter), the region cleavable by the sheddase may be present in the hinge domain, the transmembrane domain, or the other sites. Also, the extracellular hinge domain means a region between the ligand-binding site and the transmembrane domain.

The shedding structure is a structure found in the transmembrane domains of many tyrosine kinase receptors. As shown in Examples below, it was demonstrated that trogocytosis could be suppressed irrespective of the type of shedding structure as long as the shedding structure was cleavable by an in-vivo sheddase. It is inferred from this result that this is because the degree of adhesion between the receptor and the ligand therefor was reduced through shedding and thus trogocytosis was suppressed. Accordingly, the shedding structure used in the present invention may have any structure as long as the shedding structure is cleavable by an in-vivo sheddase. Examples of proteins having a shedding structure include notch proteins (specifically, notch1 protein, notch2 protein, notch3 protein, and notch4 protein) (SEQ ID NOS: 2 and 1 represent the amino acid sequence of a transmembrane domain having the shedding structure of mouse notch1 and the base sequence encoding this domain, respectively; SEQ ID NOS: 28 and 27 represent the amino acid sequence of a transmembrane domain having the shedding structure of human notch1 and the base sequence encoding this domain, respectively; and SEQ ID NOS: 33 to 36 represent sequences obtained by deleting a portion of the amino acid sequence represented by SEQ ID NO: 28), amyloid precursor proteins (SEQ ID NOS: 4 and 3 represent the amino acid sequence of a transmembrane domain having a shedding structure and the base sequence encoding this domain, respectively), CD28 (SEQ ID NOS: 22 and 21 represent the amino acid sequence of a transmembrane domain having a shedding structure and the base sequence encoding this domain, respectively), ALCAM (activated leukocyte cell adhesion molecule), CD44, VEGF receptor family (Vascular Endothelial Growth Factor Receptors: VEGFR-1, VEGFR-2, VEGFR-3, etc.), EGF (Epidermal growth factor), and EGF receptor family (e.g., ErbB1, ErbB2, ErbB3, ErbB4, etc.). The transmembrane domains derived from these proteins may be wild-type transmembrane domains (e.g., transmembrane domains that include a sequence represented by any of SEQ ID NOS:2, 4, 22, 28, and 33 to 36) or mutant transmembrane domains that include amino acid sequences having high similarity or identity to the amino acid sequences of the wild-type transmembrane domains and that are cleavable by a sheddase. The receptor of the present invention can be designed and produced by, for example, substituting the transmembrane domain of a receptor that does not have a shedding structure (e.g., transmembrane domain of CD8 (SEQ ID NOS: 6 and 5 represent the amino acid sequence of this domain and the base sequence encoding this domain, respectively)) with a transmembrane domain having a shedding structure, or substituting a portion of the extracellular domain side of the transmembrane domain or a vicinity thereof (e.g., inside of the hinge domain) with a corresponding region of a transmembrane domain having a shedding structure. The receptor of the present invention can also be designed and produced by, for example, substituting the transmembrane domain of a receptor having a shedding structure with a transmembrane domain having a different shedding structure that is more susceptible to shedding, or introducing mutation (e.g., Swe mutation of APP and the like) that further enhances the susceptibility to shedding. SEQ ID NOS: 8 and 7 represent the amino acid sequence of the transmembrane domain derived from the Swe mutant of APP and the base sequence encoding this domain, respectively.

Most of sheddases having an ability to cleave the shedding structure belong to the ADAM (a disintegrin and metalloproteinase) family or the BACE (beta-site amyloid precursor protein cleaving enzyme) family. Examples of the sheddases belonging to the ADAM family include ADAM8, ADAM9, ADAM10, ADAM12, ADAM15, ADAM17, ADAM18, ADAM19, ADAM20, ADAM21, ADAM28, ADAM30, ADAM33, and ADAMDEC1, and examples of the sheddases belonging to the BACE family include BACE1 and the like.

The transmembrane domain of the wild-type notch protein includes the S2 cleavage site cleavable by ADAM, and the S3 cleavage site and the S4 cleavage site that are cleavable by γ-secretase. The intracellular domain of the notch protein is released into the cytoplasm by the γ-secretase cleaving the S3 and S4 cleavage sites, and is then transferred to the nucleus. From the viewpoint of suppressing trogocytosis, the release of the intracellular domain into the cytoplasm is not essential to the receptor of the present invention, and therefore, the receptor of the present invention may have a function of releasing the intracellular domain into the cytoplasm or have no releasing function. Regarding this intracellular domain releasing function, in the case of the notch protein, the cleavage by the γ-secretase can be suppressed by introducing mutation into the S3 cleavage site of the transmembrane domain, and further mutation may be introduced into the S4 cleavage site. Accordingly, the receptor of the present invention may have no cleavage sites cleavable by the γ-secretase in the transmembrane domain and additionally no cleavage sites cleavable by the γ-secretase in the intracellular domain. In one aspect, the transmembrane domain of the receptor of the present invention excludes the transmembrane domain of the wild-type notch protein.

In this specification, the wording "amino acid sequence having high similarity or identity to a specific amino acid sequence" means an amino acid sequence having 80% or more, 85% or more, 90% or more, 91% or more, 92% or more, 93% or more, 94% or more, 95% or more, 96% or more, 97% or more, 98% or more, or 99% or more similarity or identity to the specific amino acid sequence, or an amino acid sequence obtained through substitution, deletion, insertion, and/or addition of one or several (e.g., 2, 3, 4, 5, 6, 7, 8, 9, or 10) amino acids in the specific amino acid sequence.

The intracellular domain of the receptor is a domain that is to be present inside the cell when the receptor is immobilized on the cell membrane via the transmembrane domain, and in contrast, a domain that is to be exposed to the outside of the cell is referred to as an "extracellular domain". The intracellular domain typically includes a signal transduction domain. As shown in Examples below, it was demonstrated that trogocytosis occurred even when a receptor that did not include a signal transduction domain was used, and therefore, the intracellular domain need not include a signal transduction domain.

The signal transduction domain is not particularly limited as long as it can transduce signals into an immune cell, and examples thereof include intracellular regions derived from one or two or more proteins selected from the group consisting of MHC class I molecules, TNF receptors, immunoglobulin-like proteins, cytokine receptors, integrin, activated NK cell receptors, Toll-like receptors, B7-H3, BAFFR, BTLA, BY55 (CD160), CD2, CD3γ, CD3δ, CD3ε, CD3ζ, CD4, CD5, CD7, CD8α, CD8β, CD11a, CD11b, CD11c, CD11d, CD18, CD19, CD19a, CD22, CD27, CD28, CD29, CD30, CD40, CD49a, CD49D, CD49f, D66d, CD69, CD79a, CD79b, CD84, CD96 (Tactile), CD103, 4-1BB (CD137), CDS, CEACAM1, CRTAM, CNAM1(CD226), DAP10, Fc receptor β chains, Fc receptor γ chains, GADS, GITR, HVEM(LIGHTR), IA4, ICAM-1, ICOS (CD278), IL2Rβ, IL2Rγ, IL7Rα, ITGA4, ITGA6, ITGAD, ITGAE, ITGAL, ITGAM, ITGAX, ITGB1, ITGB2, ITGB7, KIRDS2, Ly9 (CD229), LAT, LFA-1 (CD11a/CD18), LIGHT, LTBR, NKG2C, NKG2D, NKp30, NKp44, NKp46, NKp80 (KLRF1), OX40, PAG/Cbp, PSGL1, SELPLG (CD162), SEMA4D (CD100), SLAM (SLAMF1, CD150, IPO-3), SLAMF4 (CD244, 2B4), SLAMF6 (NTB-A, Ly108), SLAMF7, SLAMF8 (BLAME), SLP-76, TNFR2, TRANCE/RANKL, VLA1, and VLA-6 (these examples may also be referred to as "examples of a typical signal transduction domain"). Alternatively, it is also possible to use a mutant signal transduction domain that includes an amino acid sequence having high similarity or identity to the natural amino acid sequences of the signal transduction domains above. In one aspect of the invention, the signal transduction domain includes the signal transduction domain of at least one protein selected from a group consisting of the CD3ζ chain, CD28 and 4-1BB, preferably the signal transduction domains of two proteins of the group above (e.g., the signal transduction domains of CD28 and the CD3ζ chain), and more preferably the signal transduction domains of all the proteins of the group above. SEQ ID NOS: 10 and 9 represent the amino acid sequence of the signal transduction domain of the CD3ζ chain and the base sequence encoding this domain, respectively; SEQ ID NOS: 12 and 11 represent the amino acid sequence of the signal transduction domain of CD28 and the base sequence encoding this domain, respectively; and SEQ ID NOS: 14 and 13 represent the amino acid sequence of the signal transduction domain of 4-1BB and the base sequence encoding this domain, respectively.

T cells are activated by intracellular signals mediated by TCR (primary activation), and this activation is enhanced by intracellular signals mediated by costimulatory molecules (secondary activation). Accordingly, when the immune cell above is a T cell, the signal transduction domain is preferably a combination of a signal transduction domain for inducing the primary activation and a signal transduction domain for inducing the secondary activation. The signal transduction domain for inducing the primary activation may be a signal transduction domain that includes an immunoreceptor tyrosine-based activation motif (ITAM), and specific examples thereof include CD3γ, CD3δ, CD3ε, CD3ζ, Fc receptor β chains, Fc receptor γ chains, CD5, CD22, CD66d, CD79a, and CD79b, among which CD3ζ is preferable. Examples of the signal transduction domain for inducing the secondary activation include signal transduction domains other than those listed as the signal transduction domains for inducing the primary activation, out of the examples of a typical signal transduction domain. When the signal transduction domain for inducing the primary activation and the signal transduction domain for inducing the secondary activation are used in combination, the signal transduction domain includes one or more signal transduction domains for inducing the primary activation and one or more signal transduction domains for inducing the secondary activation, but the signal transduction domain of a preferable aspect includes one signal transduction domain for inducing the primary activation and a plurality of signal transduction domains for inducing the secondary activation. Both the signal transduction domain for inducing the primary activation and the signal transduction domain for inducing the secondary activation may be of the mutant type.

As shown in Examples below, the CAR having an intracellular domain in which the same (the same type of) two signal transduction domains were linked in series (in tandem) had higher antitumor activity than the CAR having one signal transduction domain. Accordingly, the receptor of the present invention may include two or more (e.g., two, three, four, five, or more) signal transduction domains of the same type (e.g., signal transduction domains of CD28 or 4-1BB). When the receptor of the present invention includes two or more intracellular domains, the intracellular domains may be linked directly or via a linker such as a linker peptide.

In this specification, the term "similarity" means the percentage (%) of identical amino acid residues and similar amino acid residues with respect to all the overlapping amino acid residues in the optimum alignment when two amino acid sequences are aligned using a mathematical algorithm known in the art (preferably, in the algorithm, introduction of gaps into one or both of the sequences can be taken into consideration for the purpose of the optimum alignment). The term "similar amino acids" means amino acids that are similar to one another in terms of physicochemical properties, and examples thereof include amino acids classified into the same group such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxy group (Ser, Thr), and amino acids with a small side chain (Gly, Ala, Ser, Thr, Met). It is anticipated that substitution with such a similar amino acid does not cause a change in the phenotype of a protein (which is known as the conservative amino acid substitution). Specific examples of the conservative amino acid substitution are well known in the art and are described in various documents (see Bowie et al., Science, 247: 1306-1310 (1990), for example). The similarity or identity of an amino acid sequence in this specification can be calculated using the homology calculation algorithm NCBI BLAST (National Center for Biotechnology Information Basic Local Alignment Search Tool) under the following conditions (expectation value = 10; gaps allowed; matrix = BLOSUM62; filtering = OFF).

The signal transduction domain is a domain having a function of transducing signals into a cell, particularly an immune cell. The "immune cell" for use in the present invention is not particularly limited as long as it is a cell (so-called immune effector cell) having an ability to possibly impair a target cell such as a cancer cell through some type of action mechanism, and examples thereof include a T cell, which plays a role in the cell-mediated immunity out of the acquired immunities, an NK cell, which plays a role in the natural immunity, a monocyte, a macrophage, a dendritic cell, and a B cell. In particular, the T cell, the natural killer cell, and the macrophage are preferable, among which the T cell is particularly preferable. In this specification, the term "T cell" means a CD3-positive cell, and examples thereof include a cytotoxic T cell (CTL) (CD8-positive cell), a helper T cell (CD4-positive cell), a regulatory T cell, an effector T cell, a γδ-T cell (usually negative for both CD8 and CD4), among which the cytotoxic T cell and the helper T cell are preferable.

In this specification, the term "modified receptor" including a modified T cell receptor, a modified Fc receptor, a modified tyrosine kinase receptor, and the like means a receptor obtained through deletion, substitution, insertion, and/or addition of one or more amino acids included in a natural receptor or known artificial receptor. A site to be modified is not particularly limited, but the transmembrane domain or a vicinity thereof (e.g., inside of the hinge domain) is typically modified.

In this specification, the term "chimeric antigen receptor (CAR)" means an artificially constructed hybrid protein that includes an antigen-binding domain (e.g., scFv) of an antibody linked to a T-cell signal transduction domain. One feature of the CAR is an ability to convert, in a non-MHC-restricted manner, the specificity and reactivity of an immune cell into those against a selected target by utilizing the antigen-binding property of a monoclonal antibody. The non-MHC-restricted antigen recognition imparts an ability to recognize an antigen irrespective of antigen processing to a CAR-expressing immune cell, and thus the main mechanism of the tumor escape is bypassed. Furthermore, the CAR has the advantage that the CAR does not form a dimer together with the endogenous TCR α and β chains when expressed in a T cell.

The CAR of the present invention includes an antigen-binding domain of an antibody that can specifically recognize the surface antigen of a target cell (e.g., a cell surface antigen of a cancer cell), a transmembrane domain, and a signal transduction domain. The CAR of the present invention may include an extracellular hinge domain.

When the receptor or CAR of the present invention includes an extracellular hinge domain, the extracellular hinge domain is composed of, for example, 1 to 300 amino acids, 5 to 100 amino acids, or 10 to 70 amino acids. The extracellular hinge domain may be composed of 6 to 29 amino acids. The extracellular hinge domain of the CAR can be, for example, a hinge domain derived from CD3, CD8, CD28, or KIR2DS2, or IgG4, IgD, or another immunoglobulin. The hinge domain and the transmembrane domain may be derived from the same protein. Alternatively, it is also possible to use a mutant hinge domain that includes an amino acid sequence having high similarity or identity to the natural amino acid sequences of the hinge domains above. In one aspect of the present invention, the hinge domain is the hinge domain of IgG4. SEQ ID NOS: 18 and 17 represent the amino acid sequence of the hinge domain of IgG4 and the base sequence encoding this domain, respectively.

It can be observed that a CAR formed by coupling a portion of the hinge domain of CD28 to the transmembrane domain also exhibits the trogocytosis suppressing effects. Accordingly, examples of the hinge domain also include the hinge domain of CD28 and a portion thereof. SEQ ID NOS: 24 and 23 represent the amino acid sequence of the hinge domain of CD28 and the base sequence encoding this domain, respectively. For example, a hinge domain that includes a portion (SEQ ID NO: 26) composed of the amino acid residues from position 19 to position 30 in the amino acid sequence of SEQ ID NO: 24 or a mutant domain that includes an amino acid sequence having high similarity or identity to the amino acid sequence of SEQ ID NO: 26 can be used as the portion of the hinge domain of CD28. The portion of the hinge domain of CD28 and the mutant thereof are typically composed of 29 or less (e.g., 25, 24, 23, 22, 21, 20, 19, 18, 17, 16, 15, 14, 13, or 12) amino acids. In addition, it is preferable that the extracellular hinge domain and the transmembrane domain are derived from the same protein (for example, both are derived from CD28). Accordingly, in one aspect of the present invention, the receptor of the present invention includes a sequence that includes the amino acid sequence of SEQ ID NO: 24 and the amino acid sequence of SEQ ID NO: 22, or an amino acid sequence having high similarity or identity to this sequence. Furthermore, in another aspect, the hinge domain of the receptor of the present invention does not include a hinge domain consisting of the amino acid sequence of SEQ ID NO: 24 and/or a region consisting of an amino acid sequence having 80% or more identity to this sequence.

In this specification, the term "T cell receptor (TCR)" means a receptor that is composed of a dimer of TCR chains (α chain, β chain, γ chain, δ chain) and recognizes an antigen or antigen-HLA (human leukocyte antigen) (MHC; major histocompatibility complex) complex to transmit stimulatory signals to a T cell. The TCR is typically composed of a dimer of the α chain and the β chain or a dimer of the γ chain and the δ chain. Each TCR chain is composed of a variable region and a constant region, and the variable region includes three complementarity determining regions (CDR1, CDR2, CDR3).

It is preferable that the constant region of the TCR chain for use in the present invention is the constant region of the natural TCR chain with a predetermined modification. This modification is, for example, substitution of a specific amino acid residue in the constant region of the natural TCR chain with a cysteine residue for the purpose of increasing the dimer expressing efficiency due to a disulfide bond between the TCR chains, but there is no limitation thereto.

The Fc receptor is a receptor having an ability to bind to the Fc region of an antibody. Examples of the Fc receptor include the Fcα receptors (e.g., FcαRI and the like) (receptors specific to IgA), the Fcγ receptors (e.g., CD64 (FCGRI), CD32 (FCGRII), CD16 (FCGRIII), and the like) (receptors for the Fc site of IgG), the Fcε receptors (e.g., FcεRI, FcεRII, and the like) (receptors capable of binding to IgE), neonatal Fc receptors (receptors capable of binding to maternal IgG), the Fc receptor-like proteins, and the Fcα/µ receptors (receptors capable of binding to IgM and IgA). Among these, the Fcγ receptors are preferable.

The tyrosine kinase receptor is also called a receptor-type tyrosine kinase, has tyrosine kinase activity, and is a cell surface receptor having a high affinity for many polypeptide-type growth factors, cytokines, and hormones. The TCR is also a tyrosine kinase receptor, but in this specification, the term "tyrosine kinase receptor" means tyrosine kinase receptors other than TCR unless otherwise stated. Examples of the tyrosine kinase receptor family include the EGF receptor family (e.g., ErbB1, ErbB2, ErbB3, ErbB4, and the like), the insulin receptor family (e.g., IR-A, IR-B, and the like), the PDGF receptor family (e.g., PDGFR-αα, PDGFR-αβ, PDGFR-ββ, and the like), the VEGF receptor family (e.g., VEGFR-1, VEGFR-2, VEGFR-3, and the like), the FGF receptor family (e.g., FGFR1, FGFR2, FGFR3, FGFR4, FGFRL1, FGFR6, and the like), the CCK family, the GF receptor family, the HGF receptor family (e.g., MET and the like), the Eph receptor family (e.g., EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, and the like), the AXL family, the TIE family, the RYK family, the DDR family, the RET family (e.g., RET51, RET43, RET9, and the like), the ROS family, the LTK family, the ROR family, the MuSK family, the LMR family, and other families. The tyrosine kinase receptor may belong to any of these families. In addition, examples of the tyrosine kinase receptor also include CD3, and costimulatory molecules (e.g., CD28, 4-1BB, CD357, CD40, ICOS, OX40, and the like).

The origin of the receptor for use in the present invention is not particularly limited, and the receptor is preferably derived from a mammal (e.g., a human, a rat, a mouse, a guinea pig, a rabbit, a sheep, a horse, a pig, a cow, a dog, a cat, a monkey, or the like). In particular, the receptor is more preferably derived from a human.

The receptor of the present invention can be produced through a genetic engineering technique using a nucleic acid or vector of the present invention, which will be described later. For example, when the receptor is a monomer such as the CAR, the receptor can be produced by introducing a nucleic acid encoding the receptor into a cell, allowing the receptor to be expressed in the cell, and isolating the receptor. When the receptor is a dimer such as the TCR, the receptor can be produced by, for example, introducing both a nucleic acid encoding one of the polypeptides included in the receptor of the present invention and a nucleic acid encoding the other polypeptide into a cell, allowing the polypeptides to be expressed and form a dimer, and isolating the dimer using a method known per se. The same applies to a case where the receptor is a polymer composed of three or more monomers.

### 2. Nucleic Acid Encoding Receptor of the Present Invention and Vector Including the Same

The present invention provides a nucleic acid encoding the above-described receptor of the present invention (the nucleic acid may also be referred to as the "nucleic acid of the present invention" hereinafter). Regarding the nucleic acid of the present invention, when the receptor is a dimer, a nucleic acid encoding one of the polypeptides included in the receptor of the present invention and a nucleic acid encoding the other polypeptide may be included in separate molecules, or both of the nucleic acids encoding the respective polypeptides may be included in one molecule. The same applies to a case where the receptor is a polymer composed of three or more monomers.

The nucleic acid of the present invention may be DNA or RNA, or a DNA/RNA chimera, among which DNA is preferable. The nucleic acid may be double-stranded or single-stranded. When the nucleic acid is double-stranded, it may be double-stranded DNA, double-stranded RNA, or a DNA:RNA hybrid. When the nucleic acid is RNA, "T" in the base sequence is read as "U" in the RNA sequence. The nucleic acid of the present invention may include a natural nucleotide, a modified nucleotide, a nucleotide analogue, or a mixture thereof as long as a polypeptide can be expressed in vitro or in a cell.

The nucleic acid of the present invention can be produced using a method known per se, and can be cloned in accordance with the following procedure, for example: based on known DNA sequence information of a receptor, an oligo DNA primer for covering a desired portion of the sequence is synthesized, and total RNA or an mRNA fraction prepared from a cell with the sequence is used as a template and is amplified through the RT-PCR method. Alternatively, DNA encoding the entire length or a portion can be constructed by chemically synthesizing a DNA chain or connecting synthesized partially overlapping oligo DNA short chains through the PCR method (overlap PCR method) or the Gibson Assembly method. In addition, mutation can also be introduced into the sequence as appropriate.

The nucleic acid of the present invention can be incorporated into an expression vector. Therefore, the present invention provides an expression vector that comprises the above-described nucleic acid of the present invention (this vector may also be referred to as the "vector of the present invention" hereinafter).

Examples of a promoter used in the vector of the present invention include a ubiquitin promoter, an EF1α promoter, a CAG promoter, an SRα promoter, an SV40 promoter, an LTR promoter, a CMV (cytomegalovirus) promoter, an RSV (Rous sarcoma virus) promoter, an MoMuLV (Moloney murine leukemia virus) LTR, and an HSV-TK (herpes simplex virus thymidine kinase) promoter. In particular, the ubiquitin promoter, the EF1α promoter, the CAG promoter, the MoMuLVLTR, the CMV promoter, and the SRα promoter are preferable.

The vector of the present invention may optionally include a transcriptional regulatory sequence, a translational regulatory sequence, a ribosome-binding site, an enhancer, a replication origin, a poly-A addition signal, a selection marker gene, and the like in addition to the promoter above. Examples of the selection marker gene include a dihydrofolate reductase gene, a neomycin-resistant gene, and a puromycin-resistant gene.

When the receptor is a dimer, an expression vector that includes a nucleic acid encoding one of the polypeptides included in the receptor of the present invention above and a nucleic acid encoding the other polypeptide is introduced into a target cell, and thus a heterodimer composed of the two polypeptides can be formed inside the cell or on the surface of the cell. In this case, the nucleic acid encoding one of the polypeptides included in the receptor of the present invention and the nucleic acid encoding the other polypeptide may be incorporated into separate expression vectors or one expression vector. When incorporated into one expression vector, these two types of nucleic acids are preferably incorporated thereinto with a sequence that enables polycistronic expression being therebetween. Using the sequence that enables polycistronic expression makes it possible to more efficiently express a plurality of genes incorporated into one type of expression vector. Examples of the sequence that enables polycistronic expression include 2A sequences (e.g., the 2A sequence (F2A) derived from a foot- and-mouth disease virus (FMDV), the 2A sequence (E2A) derived from an equine rhinitis A virus (ERAV), the 2A sequence (P2A) derived from a porcineteschovirus (PTV-1), and the 2A sequence (T2A) derived from a Thosea asigna virus (TaV)) (PLoS ONE, 3:e2532, 2008, Stem Cells 25, 1707, 2007, and the like), and internal ribosome entry sites (IRESs) (U.S. Patent No. 4,937,190), and the 2A sequences are preferable from the viewpoint of a uniform expression level. Among the 2A sequences, the P2A sequence and the T2A sequence are preferable. The same applies to a case where the receptor is a polymer composed of three or more monomers.

Examples of the expression vector that can be used in the present invention include viral vectors and plasmid vectors. Examples of the viral vectors include retroviral vectors (that includes lentiviral vectors and pseudo-type vectors), adenoviral vectors, adeno associated viral vectors, herpes viral vectors, Sendai viruses, and episomal vectors. A transposon expression system (e.g., PiggyBac system) may also be used. Examples of the plasmid vectors include animal cell expression plasmids (e.g., pa1-11, pXT1, pRc/CMV, pRc/RSV, and pcDNAI/Neo) and the like.

### 3. Immune Cell Having Receptor of the Present Invention and Medicine Comprisingthe Same

The present invention provides an immune cell having the nucleic acid or vector of the present invention (this immune cell may be referred to as the "immune cell of the present invention" hereinafter) and a medicine comprising the immune cell of the present invention (this medicine may be referred to as the "medicine of the present invention" hereinafter). It is preferable that the immune cell of the present invention expresses the receptor of the present invention on the cell surface. The immune cell of the present invention may express one type of receptor of the present invention or two or more types of receptors (e.g., a combination of the CAR and the TCR) of the present invention.

The immune cell or medicine of the present invention can be administered to a mammal (e.g., a human, a rat, a mouse, a guinea pig, a rabbit, a sheep, a horse, a pig, a cow, a dog, a cat, a monkey, or the like). Accordingly, a method for treating or preventing a cancer in a mammal that includes administering the immune cell or medicine of the present invention to the mammal is also provided. A therapeutic or preventive medicine for a cancer also encompasses a medicine capable of treating and preventing the disease unless otherwise stated. The same applies to a method for treating or preventing a cancer.

In this specification, the "therapeutic medicine" includes not only a medicine intended for the radical treatment of a cancer but also, for example, a medicine intended for suppression of cancer progression, a medicine intended for alleviation of symptoms (e.g., improvement into minimal manifestations (MM), which does not interfere with life and work), or a medicine for alleviate an aftereffect. For example, a cancer is a disease that progresses over a long period of time (typically in years), and therefore, early initiation of treatment can prevent the progression of symptoms. In addition, in this specification, the "preventive medicine" includes not only a medicine intended to reduce the risk of development of a cancer in a subject that have not developed a cancer but also a medicine intended to reduce the risk of recurrence of a cancer in a subject that have developed the cancer. The same applies to a method for treating or preventing a cancer.

The immune cell having the nucleic acid or vector of the present invention may be the same cells as the immune cells listed in "1. Artificial Receptor with Transmembrane Domain Having Shedding Structure" above, but is preferably a T cell and more preferably a cytotoxic T cell. The immune cell expressing the receptor of the present invention can be obtained by introducing the nucleic acid or vector of the present invention into an immune cell (e.g., peripheral blood T cell) collected from a living body or an immune cell obtained by inducing differentiation of a progenitor cell of the immune cell or a pluripotent stem cell. Alternatively, the immune cell expressing the receptor of the present invention may also be obtained by introducing the nucleic acid or vector of the present invention into a progenitor cell of a target immune cell or a pluripotent stem cell and differentiating the cell into the target immune cell using a differentiation inducing method known per se.

The immune cell can be collected from, for example, peripheral blood, bone marrow, and cord blood of, for example, a human or non-human mammal. When the immune cell expressing the receptor of the present invention is used to treat or prevent a cancer, it is preferable to collect the immune cell from a subject themself to be treated or a donor with the same HLA type as that of the subject to be treated.

Examples of the progenitor cells of the immune cells include hematopoietic stem cells, lymphoid stem cells, multipotent progenitors (MMPs) that have lost the self-replicating ability, common myeloid-lymphoid progenitors (MLP), myeloid progenitors (MPs), granulocyte mononuclear progenitors (GMPs), macrophage-dendritic cell progenitors (MDPs), dendritic cell progenitors (DCPs), and T progenitors (e.g., DN1 cells, DN2 cells, DN3 cells, DN4 cells, DP cells, etc.).

Examples of the pluripotent stem cells include embryonic stem cells (ES cells), induced pluripotent stem cells (iPS cells), embryonic carcinoma cells (EC cells), and embryonic germ cells (EG cells). When the pluripotent stem cells are ES cells or arbitrary cells derived from human embryos, the cells may be obtained by destruction of the embryo or they may be obtained without destruction of the embryo, but preferably they are cells obtained without destruction of the embryo.

In this specification, the term "pluripotent stem cell" means an embryonic stem cell (ES cell) and a cell that potentially has a pluripotent differentiation ability similar to that of the ES cell (i.e., a cell that potentially has an ability to differentiate into various biological tissues (all of the endoderm, the mesoderm, and the ectoderm)). An example of the cell having a pluripotent differentiation ability similar to that of the ES cell is an "induced pluripotent stem cell (iPS cell or iPSC)".

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292: 154-156), and ES cell lines were later established in primates including humans and monkeys (J.A. Thomson et al. (1998), Science 282: 1145-1147; J.A. Thomson et al. (1995), Proc. Natl. Acad. Sci. USA, 92: 7844-7848; J.A. Thomson et al. (1996), Biol. Reprod., 55: 254-259; J.A. Thomson and V.S. Marshall (1998), Curr. Top. Dev. Biol., 38: 133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

The ES cell line used for the present invention may be human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines, etc., distributed by ESI Bio Co., H1 and H9 lines, etc., distributed by WiCell Research, KhES-1 line, KhES-2 line, KhES-3 line, KhES-4 line, KhES-5 line, SSES1 line, SSES2 line and SSES3 lines, etc., distributed by Riken.

iPS cells are cells obtained by reprogramming of mammalian somatic cells or undifferentiated stem cells by introduction of specific factors (nuclear reprogramming factors). A large number of iPS cells currently exist, among which there may be used human iPSCs established by introduction of the 4 factors Oct3/4·Sox2·Klf4·c-Myc into human fibroblasts by Yamanaka (Takahashi K, Yamanaka S., et al. Cell, (2007) 131: 861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5): 409-12, Okita K et al. Stem Cells. 31(3): 458-66.), and so on. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451: 141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y, Ding S., et al., Cell Stem Cell, (2008) Vol.3, Issue 5, 568-574;, Kim JB., Scholer HR., et al., Nature, (2008) 454, 646-650; Huangfu D., Melton, DA., et al., Nature Biotechnology, (2008) 26, No 7, 795-797), or patents (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A line, HiPS-RIKEN-2A line, HiPS-RIKEN-12A line, Nips-B2 line, etc., by Riken, 253G1 line, 253G4 line, 1201C1 line, 1205D1 line, 1210B2 line, 1383D2 line, 1383D6 line, 201B7 line, 409B2 line, 454E2 line, 606A1 line, 610B1 line, 648A1 line, 1231A3 line, FfI-01s04 line, etc., by Kyoto University, with the 1231A3 line being preferred.

In this specification, the term "hematopoietic progenitor cell" means a multipotent stem cell that can differentiate into hemocyte cells. In humans, the hematopoietic progenitor cell is mainly present in bone marrow and differentiates into a leukocyte (a neutrophil, an eosinophil, a basophil, a lymphocyte, a monocyte, a macrophage), an erythrocyte, a platelet, a mast cell, and dendritic cells. In the present invention, the term "hematopoietic progenitor cell" means a CD34-positive cell and preferably a CD34/CD43-double-positive (DP) cell. The origin of the hematopoietic progenitor cell used in the present invention is not limited, and may be, for example, a hematopoietic progenitor cell obtained by inducing differentiation of a pluripotent stem cell using a method described below, or a hematopoietic progenitor cell isolated from a living tissue using a known method.

A method for differentiating a pluripotent stem cell into an immune cell such as a T cell may be, for example, a method that includes (1) a step of differentiating a pluripotent stem cell into which the nucleic acid or vector of the present invention has been introduced into a hematopoietic progenitor cell and (2) a step of differentiating the hematopoietic progenitor cell into an immune cell. The step (1) above may be, for example, a method of culturing a pluripotent stem cell in a culture medium for induction to a hematopoietic progenitor cell as described in WO 2013/075222, WO 2016/076415, Liu S. et al., Cytotherapy, 17 (2015); 344-358, and the like. When the immune cell is a T cell, the step (2) above may be, for example, a method that includes (2-1) a step of inducing a CD4CD8-double-positive T cell from the hematopoietic progenitor cell and (2-2) a step of inducing a CD8-positive T cell from the CD4CD8-double-positive T cell as described in WO 2016/076415 and the like.

The culture medium for induction to a hematopoietic progenitor cell is not particularly limited, but can be prepared using a culture medium for use in animal cell culture as a basal culture medium. Examples of the basal culture medium include the Iscove's Modified Dulbecco's Medium (IMDM) culture medium, the Medium 199 culture medium, the Eagle's Minimum Essential Medium (EMEM) culture medium, the αMEM culture medium, the Dulbecco's modified Eagle's Medium (DMEM) culture medium, the Ham's F12 culture medium, the RPMI 1640 culture medium, the Fischer's culture medium, Neurobasal Medium (Life Technologies Corporation), and mixed culture mediums obtained by mixing these culture mediums. The culture medium may contain a serum or may be a serum-free culture medium. The basal culture medium may contain, for example, vitamin C (e.g., ascorbic acid), albumin, insulin, transferrin, selenium, fatty acids, trace elements, 2-mercaptoethanol, thiol glycerol, lipids, amino acids, L-glutamine, non-essential amino acids, vitamins, growth factors, low-molecular-weight compounds, antibiotics, antioxidants, pyruvic acid, buffers, inorganic salts, cytokines, etc. as necessary.

The culture medium for inducing differentiation into a CD4CD8-double-positive T cell for use in the step (2-1) is not particularly limited, but can be prepared using a culture medium for use in animal cell culture as a basal culture medium. Examples of the basal culture medium include the same basal culture media as those used in the step (1) above. The culture medium may contain a serum or may be a serum-free culture medium. The basal culture medium may contain, for example, vitamin C, albumin, insulin, transferrin, selenium, fatty acids, trace elements, 2-mercaptoethanol, thiol glycerol, lipids, amino acids, L-glutamine, non-essential amino acids, vitamins, growth factors, low-molecular-weight compounds, antibiotics, antioxidants, pyruvic acid, buffers, inorganic salts, cytokines, etc. as necessary.

Examples of the basal culture medium and culture medium additives for use in the step (2-2) include the same basal culture media and culture medium additives as those described in the step (1). The culture medium above may contain an adrenocortical hormone agent. Examples of the adrenocortical hormone agent include a glucocorticoid and its derivatives, and examples of the glucocorticoid include cortisone acetate, hydrocortisone, fludrocortisone acetate, prednisolone, triamcinolone, methylprednisolone, dexamethasone, betamethasone, and beclomethasone propionate. In particular, dexamethasone is preferable.

There is no particular limitation on the method of introducing the nucleic acid or vector of the present invention into a cell, and a known method can be used. A nucleic acid or plasmid vector can be introduced using, for example, calcium phosphate coprecipitation, a PEG method, electroporation, microinjection, lipofection, or the like. For example, methods described in Cell Technology Extra Issue 8: New Cell Technology Experimental Protocols, 263-267 (1995) (published by Shujunsha Co., Ltd.); Virology, Vol. 52, 456 (1973); Folia Pharmacol. Jpn., Vol. 119 (No. 6), 345-351 (2002); and the like can be used. A viral vector can be introduced into a cell by introducing the nucleic acid of the present invention into a proper packaging cell (e.g., Plat-E cell) or complementary cell line (e.g., 293 cell), collecting the viral vector produced into the culture supernatant, and infecting a cell with the vector using an appropriate method depending on the viral vector. For example, specific means of using a retroviral vector as the vector are disclosed in WO 2007/69666; Cell, 126, 663-676 (2006); Cell, 131, 861-872 (2007); and the like. In addition, specific means of using a lentivirus as the vector are disclosed in Zufferey R. et al., Nat Biotechnol, 15(9): 871-895 (1997), and the like. In particular, when a retroviral vector is used, using a recombinant fibronectin fragment CH-296 (manufactured by Takara Bio Inc.) makes it possible to efficiently introduce genes into various types of cells. Alternatively, the nucleic acid or vector of the present invention may be introduced into the genome of a cell through genome editing (e.g., the CRISPR/Cas system, TALEN, ZFN, etc.).

The nucleic acid of the present invention may also be introduced directly into a cell in the form of RNA and used to express the receptor of the present invention in the cell. RNA can be introduced using a known method, and, for example, lipofection, electroporation, or the like can be favorably used.

The expression of the receptor of the present invention can be detected or measured using, for example, an immunological technique in which an antibody that recognizes a portion (e.g., the constant region of the TCR chain, and the like) of the receptor of the present invention is used. Examples of the immunological technique include an antibody array, flow cytometry analysis, radioimmunoassay (RIA), ELISA, Western blotting, immunohistostaining, enzyme immunoassay (EIA), fluorescence immunoassay (FIA), and immunochromatography.

The present invention also provides a method of producing a cell that includes a step of introducing the nucleic acid or vector of the present invention into the cell. The cells into which the nucleic acid or vector of the present invention is introduced, the introduction method, and the like are as described above. It is preferable that the cell expresses the receptor of the present invention. The expression of the receptor of the present invention can be detected or measured using the method described above. In yet another aspect, an immune cell obtained using the production method above is also provided.

The medicine of the present invention can target any cancers as long as the cancer tissues include a cancer cell expressing a cancer antigen targeted by the receptor of the present invention. Examples of the cancers include cancers such as an adenocarcinoma, a squamous cell carcinoma, an adenosquamous carcinoma, an anaplastic carcinoma, a large cell carcinoma, a small cell carcinoma, a skin cancer (e.g., melanoma and a Merkel-cell carcinoma), a breast cancer, a prostate cancer, a bladder cancer, a vaginal cancer, a neck cancer, a head and neck cancer, a uterine cancer, a cervical cancer, a liver cancer, a kidney cancer, a pancreatic cancer, a spleen cancer, a lung cancer, a non-small cell lung cancer, a tracheal cancer, a bronchial cancer, a colon cancer, a rectal cancer, a small intestinal cancer, a large intestinal cancer, a stomach cancer, an esophageal cancer, a gallbladder cancer, a testicular cancer, an ovarian cancer, a fallopian tube cancer, and a nasopharynx cancer; cancers in the bone tissue, the cartilage tissue, the adipose tissue, the muscular tissue, the vascular tissue, and the hematogenous tissue; sarcomas such as a chondrosarcoma, an Ewing's sarcoma, a rhabdomyosarcoma, a malignant hemangioendothelioma, a malignant schwannoma, an osteosarcoma, a soft tissue sarcoma; blastomas such as a hepatoblastoma, a medulloblastoma, a nephroblastoma, a neuroblastoma, a pancreatic blastoma, a pleuropulmonary blastoma, and a retinoblastoma; germ-cell tumors; a lymphoma; a leukemia, an acute myeloid leukemia, and a multiple myeloma.

Examples of components other than the immune cell that can be contained in the medicine of the present invention include pharmaceutically acceptable additives, and more specific examples thereof include a saline, a buffered saline, a cell culture medium, dextrose, water for injection, glycerol, ethanol, a stabilizer, a solubilizer, a surfactant, a buffer, a preservative, an isotonic agent, a filler, and a lubricant.

Examples of a method for administering the immune cell or medicine of the present invention include an intratumoral injection, an intravenous injection, an arterial injection, a intramuscular injection, a subcutaneous injection, and an intraperitoneal injection.

The amount of the immune cell of the present invention contained in the medicine of the present invention can be appropriately adjusted depending on the type, application, dosage form, and targeted therapeutic effect of the immune cell in consideration of, for example, the type, position, and severity of a cancer, and the age, weight, and condition of a subject to be treated. For example, the medicine of the present invention can be produced such that the number of the immune cells of the present invention to be administered per dose is typically 1×10⁴ to 1×10¹⁰, preferably 1×10⁵ to 1×10⁹, and more preferably 5×10⁶ to 5×10⁸.

The administration interval of the immune cell or medicine of the present invention is not particularly limited and can be adjusted as appropriate in consideration of the amount of the immune cell of the present invention to be administered per dose. However, the immune cell or medicine of the present invention can be independently administered, for example, four times a day, three times a day, twice a day, once a day, every other day, every three days, every four days, every five days, every six days, once a week, every eight days, every nine days, every ten days, twice a week, once a month, or twice a month.

The immune cell or medicine of the present invention can be used together with a known cancer therapeutic medicine. Examples of the cancer therapeutic medicine include alkylating agents such as cyclophosphamide, bendamustine, ifosfamide, and dacarbazine; antimetabolites such as pentostatin, fludarabine, cladribine, methotrexate, 5-fluorouracil, 6-mercaptopurine, and enocitabine; molecular target drugs such as rituximab, cetuximab, and trastuzumab; kinase inhibitors such as imatinib, gefitinib, erlotinib, afatinib, dasatinib, sunitinib, and trametinib; proteasome inhibitors such as bortezomib; calcineurin inhibitors such as ciclosporin and tacrolimus; anticancer antibiotics such as idarubicin, doxorubicin, and mitomycin C; plant alkaloids such as irinotecan and etoposide; platinum-based drugs such as cisplatin, oxaliplatin, and carboplatin; hormone therapy drugs tamoxifen and bicalutamide; immune regulating agents such as interferon, nivolumab, and pembrolizumab; and the like.

### 4. Method for Designing or Producing Receptor with Regulated Trogocytosis Effect

As shown in Examples below, it was demonstrated that the trogocytosis effect of the receptor could be regulated according to the presence or absence of the shedding structure of the receptor, the intensity of the shedding, the size of the intracellular domain, and/or the presence or absence of the signal transduction domain. More specifically, as shown in Examples below, it was found that the trogocytosis was suppressed by introducing the shedding structure into the transmembrane domain that did not have the shedding structure and the trogocytosis suppressing effects were enhanced by further introducing mutation more susceptible to shedding. Also, it was found that it was not essential for the occurrence of the trogocytosis that the receptor had the signal transduction domain, the intensity of the trogocytosis varied depending on the size of the intracellular domain, and the signal transduction domain more strongly induced the trogocytosis than a non-signal transduction domain when these domains had the same size.

Therefore, in yet another aspect, the present invention provides a method for designing a receptor with regulated trogocytosis effect or a nucleic acid encoding the receptor (this method may be referred to as the "designing method of the present invention" hereinafter), comprising
(1) a step of selecting a receptor that includes a ligand-binding site, a transmembrane domain, and a signal transduction domain into an immune cell, and
(2A) a step of adjusting sensitivity of the transmembrane domain selected in the step (1) to shedding, and/or
(2B) a step of adding, inserting, and/or deleting one or more amino acid residues in the signal transduction domain of the receptor prepared in the step (1), and/or substituting one or more amino acids in the signal transduction domain.

Also provided is a method for producing a receptor with regulated trogocytosis effect or a nucleic acid encoding the receptor (this method may be referred to as the "production method of the present invention" hereinafter), comprising
(I) a step of preparing a receptor that includes a ligand-binding site, a transmembrane domain, and a signal transduction domain into an immune cell, and
(IIA) a step of adjusting sensitivity of the transmembrane domain prepared in the step (I) to shedding, and/or
(IIB) a step of adding, inserting, and/or deleting one or more amino acid residues in the signal transduction domain of the receptor prepared in the step (I), and/or substituting one or more amino acid residues in the signal transduction domain. In the description below, when items applied to both the designing method of the present invention and the production method of the present invention are referred to, these methods may be collectively referred to as the "method of the present invention".

In this specification, the wording "with regulated trogocytosis effect" means that, compared with the case where a receptor prepared in the step (1) or (I) (this receptor may be referred to as the "target receptor" hereinafter) is introduced into an immune cell, when a receptor after implementation of the steps (2A), (2B), (IIA), and/or (IIB) of the method of the present invention (i.e., a receptor designed or produced through these steps) (this receptor may be referred to as the "receptor after implementation of the present invention" hereinafter) is introduced into an immune cell, the intensity of the trogocytosis alters (i.e., decreases or increases), or it is expected that the intensity of the trogocytosis will alter.

The receptor prepared in the step (1) or (I) of the method of the present invention is not particularly limited, but examples thereof include the known CARs, TCRs, Fc receptors, and tyrosine kinase receptors. All the contents described in "1. Artificial Receptor with Transmembrane Domain Having Shedding Structure" above are applied to these specific examples, the structure, and the like of the receptor.

When the transmembrane domain of the target receptor does not have a shedding structure, the step (2A) or (IIA) of the method of the present invention can be conducted by, for example, substituting the transmembrane domain with a transmembrane domain having a shedding structure, or substituting a portion of the extracellular domain side of the transmembrane domain or a vicinity thereof (e.g., inside of the hinge domain) with a corresponding region of a transmembrane domain having a shedding structure. Alternatively, it is also possible to cause the shedding by adding a structure (e.g., hinge domain of CD28) that can impart an ability to undergo shedding, a portion thereof, or a mutant thereof to the transmembrane domain. When the transmembrane domain of the target receptor has shedding structure, the step (2A) or (IIA) of the method of the present invention can be conducted by, for example, substituting the transmembrane domain of a receptor having a shedding structure with a transmembrane domain that does not have a shedding structure, deleting the hinge domain or substituting the hinge domain with another hinge domain, substituting the transmembrane domain with a transmembrane domain having a shedding structure with different sensitivity to shedding, or introducing mutation (e.g., Swe mutation of APP and the like) that changes the sensitivity to shedding into the transmembrane domain. It is reported that when, in the sheddase-cleavable sequence, about half of amino acid residues in a sequence near the extracellular domain side are amino acid residues with a negative charge or a sequence near the extracellular domain is glycosylated, shedding can be inhibited (Iwagishi R, et al., J Biol Chem. 295(35): 12343-12352 (2020)). Therefore, it is also possible to regulate the sensitivity to shedding by adjusting the charges or the degree of glycosylation of a sequence near the extracellular domain side in the sheddase-cleavable sequence.

When an amino acid residue is added, inserted, and/or deleted in the signal transduction domain of the target receptor in the step (2B) or (IIB) of the method of the present invention, addition of an amino acid and the like are typically conducted so as to obtain a signal transduction domain that includes an amino acid sequence having high similarity or identity to the amino acid sequence of the unmodified signal transduction domain for the purpose of retaining the signal transduction function even after such modifications. In Examples below, it was shown that trogocytosis was further suppressed by increasing the number of signal transduction domains in the receptor that included the transmembrane domain having a shedding structure. Accordingly, the step (2B) or (IIB) of the method of the present invention may be a step of changing the order or number of the intracellular domains of the target receptor. When the number of the intracellular domains is changed, it is preferable to arrange the identical intracellular domains in tandem. Furthermore, in Examples below, it was observed that trogocytosis was enhanced even when EGFP (SEQ ID NO: 20) having a length of about 240 amino acids was added to the signal transduction domain. Accordingly, when the signal transduction domain of the target receptor is substituted with a non-signal transduction domain, or a non-signal transduction domain is further added to the signal transduction domain, the upper limit of the size of the non-signal transduction domain for the substitution or addition is not particularly limited, but may be, for example, a length of 500 amino acids, 400 amino acids, 300 amino acids, 250 amino acids, 200 amino acids, 150 amino acids, or 100 amino acids.

In the steps (2A) and (2B) of the designing method of the present invention, it is not necessary to actually make a receptor, and it is sufficient that the receptor is imagined conceptually. However, such an image of the receptor is typically embodied on paper or in a program (e.g., a graphic design tool, office software, etc.) running on an electronic computer. Therefore, the act of designing a receptor that is expected to alter the intensity of trogocytosis and summarizing such receptors in the form of a table also falls under the implementation of the designing method of the present invention. Also, in the steps (IIA) and (IIB) of the production method of the present invention, it is not necessary to actually produce a receptor, and it is sufficient that a nucleic acid is produced that encodes a receptor with a modified shedding structure and/or a modified signal transduction domain and that is designed such that the receptor is expressed in a cell.

Theoretically, by conducting the steps (2A), (2B), (IIA), and/or (IIB) of the method of the present invention on the target receptor, a receptor having regulated trogocytosis effect compared with the target receptor when being introduced into a cell is designed or produced. However, it may be actually checked if the trogocytosis effect is regulated in the receptor after implementation of the present invention, and the intensity of the trogocytosis in the receptor after implementation of the present invention may be evaluated. The intensity of the trogocytosis can be evaluated, for example, using the following method as described in Examples below, but there is no limitation thereto. Immune cells (1×10⁵) expressing the target receptor or the receptor after implementation of the present invention and target cells are cocultured at a ratio of 1:1 in a 48-well plate at 37°C overnight, and then the expression level of a ligand targeted by the receptor is measured through flow cytometry using an antibody against the ligand. The expression levels of the ligand in the cells are compared, and when there are differences, it can be determined that the trogocytosis effect was regulated.

It is also possible to regulate trogocytosis by introducing a receptor designed or produced using the method of the present invention or a nucleic acid encoding the receptor into an immune cell or a cell (e.g., cancer cell) that induces trogocytosis together with an immune cell. For example, by using genome editing or the like to substitute at least one (preferably all) of nucleic acids encoding the target receptor present in a cell with a nucleic acid encoding a receptor designed or produced using the method of the present invention or insert the nucleic acid encoding a receptor designed or produced using the method of the present invention into the genome of the cell, the target receptor present in the cell is substituted or the introduced receptor competitively functions, for example, and thus it is also possible to regulate trogocytosis.

Hereinafter, the present invention will be described more specifically by way of examples, but the present invention is not limited to these examples by any means.

### Examples

### <Materials and Methods>

### Vector Construction and Viral Production

A CAR vector was constructed with a common molecular biological technique using a clinically used FCM63 CD19-specific scFv (SEQ ID NOS: 15 and 16); any of the transmembrane domain (SEQ ID NOS: 5 and 6) of CD8 as Normal-TM and the transmembrane domain of Notch1 (SEQ ID NOS: 1 and 2) and the transmembrane domains (including a portion of the extracellular domain of each protein) (SEQ ID NOS: 3 and 4, and SEQ ID NOS: 7 and 8) of APP proteins as Cleavable-TM; and any of the intracellular domain (SEQ ID NOS: 11 and 12) of CD28, the intracellular domain (SEQ ID NOS: 13 and 14)) of 4-1BB, the intracellular domain (SEQ ID NOS: 9 and 10) of the CD3ζ chain, and intracellular domains formed by using these intracellular domains in combination as the intracellular domain. The FCM63 CD19-specific scFv and the transmembrane domain of CD8 were linked to each other via the hinge domain (SEQ ID NOS: 17 and 18) of IgG4. On the other hand, the FCM63 CD19-specific scFv and Cleavable-TM were linked to each other without the hinge domain of IgG4 therebetween. A virus solution for gene transfer was produced using 293T cells, a packaging vector, and an envelope vector. The other CARs were produced in the same manner. SEQ ID NOS: 21 and 22 represent the sequence of the transmembrane domain of CD28 that includes a hinge domain (SEQ ID NOS: 25 and 26) composed of 12 amino acid residues, SEQ ID NOS: 27 and 28 represent the sequence of hCleavable (transmembrane domain that includes a portion of the extracellular domain of human Notch1), SEQ ID NOS: 29 and 30 represent the sequence of aTRBV12 ScFv, and SEQ ID NOS: 31 and 32 represent the sequence of Her2 ScFV. SEQ ID NOS: 33 to 36 represent hCleavables of 19hL31447 2828z, 19hL31448 2828z, 19hL31530 2828z, and 19hL31560 2828z, respectively.

### Detection of CAR Signals by NFAT-Jurkat

CAR-NFAT-Luc Jurkat cells were produced by introducing each CAR construct into NFAT-Luc jurkat cells (Promega J1621) via lentiviruses. After these cells and a cell line (NALM6, Raji) expressing a CD19 target were cocultured at a ratio of 1:1 (5×10⁴ cells) for a short period of time (3 h) or a long period of time (4 days), a recommended amount of luciferin (VivoGlo Promega) was added to each well, and the active intensity of NFAT signal was detected as the luminescence level using Nivo (trademark) (PerkinElmer Japan G.K.).

### T cell Stimulation and Gene Transfer

Peripheral blood derived from healthy humans was obtained in accordance with the experimental plan approved by the Ethics Committee of Kyoto University. Peripheral blood T cells (PBMCs) were isolated through a centrifugal concentration gradient technique using Leucosep (trademark) (Greiner Bio-One Co. Ltd.). A recommended amount of Dynabeads Human T-Activator CD3/CD28 (VERITAS) was added to the isolated PBMCs, and the cells were cultured and stimulated in a culture medium produced by adding IL15 and IL-7 to a CD15% FBS + a-MEM medium. Retroviruses were added on the second and third days of the stimulation to introduce each CAR gene. Dynabeads were removed on the third day of the stimulation, followed by addition of the culture medium and proliferation of CAR-PBMCs.

### Mouse Tumor Model

8- to 12-week-old NSG mice (Jackson) were used in accordance with the experimental plan approved by the Animal Experiment Committee of Kyoto University based on the animal ethics. 5×10⁵ NALM-6 cells were administered into the tail vein through an intravenous injection on Day 0 to produce a mouse tumor model. On Days 4 and 14, 2×10⁵ CAR-T cells were intravenously administered to produce a treatment model.

### Trogocytosis Assay through Flow Cytometry

1×10⁵ CAR-T cells or CAR-jurkat cells and NALM-6 cells were cocultured at a ratio of 1:1 in a 48-well plate at 37°C overnight. After the cells were collected in a FACS buffer, various antibodies were added thereto, and the cells were stained for 30 min and were then measured using FACSAria (BD). The data was analyzed using Flowjo software. Regarding the metalloprotease inhibitor experiment, GI254023X and DAPT (Sigma) were added at various concentrations during the coculture, and then the experiment was conducted.

### Cytotoxicity Assay

The CAR-T cells and target cells were mixed at various Effector/Target ratios, and the cytotoxicity was measured in Nivo (PerkinElmer Japan G.K.) using the N-SPC kit (TECHNO SUZUTA Co., Ltd.) as recommended.

### Imaging Analysis

After NALM6 cells and jurkat cells into which CARs formed by adding Hibit-tag proteins to the termini of various CARs (19-28bbz or Cleavable-19-28bbz) had been introduced were cocultured overnight, fixation and cell membrane permeabilization were conducted using the intracellular staining kit available from Biolegend, and intracellular staining was conducted in accordance with Biolegend intracellular Flow Cytometry Staining Protocol. After the cells were stained with Mouse Anti-Hibit mAb (Promega), AlexaFluor647 goat antimouse igG (Invitrogen), and the CD19-FITC antibody, imaging analysis was conducted using image-Stream (MKII).

### Statistical Analysis

In-vitro experiments performed using cultured cells were implemented a plurality of times on all groups (n=3), and the reproducibility was examined. Excel was used for data analysis, and the significant test was conducted using a T test to compare the average values of the groups, and the significant difference was indicated using * (p<0.05), ** (p<0.01), or *** (p<0.001). In in-vivo experiments, each group was composed of five mice, survival curves were prepared using Prism, and the survival time differences were tested using a log-rank test.

### Example 1: Examination of Impact of Shedding on CAR Effects

The investigation was started based on the hypothesis that the CAR reactivity changes due to introduction of a structure capable of undergoing shedding (Cleavable-CAR) compared with a normal CAR (Normal-CAR) having a CD8 transmembrane domain that does not undergo shedding. CARs in which the transmembrane domain was changed to the transmembrane domain derived from Notch protein, which is well known as the shedding structure, were produced, and those in which the CAR reactivity was retained were determined (FIG. 1-a). In order to detect short-term reactivity with target cells in CARs having various intracellular domains, various CAR genes were expressed in NFAT-Jurkat cells, the NFAT-Jurkat cells were cocultured with NALM6 cells for 3 h, and then the luciferase expression was detected. As a result, it was found that the reactivity of the Cleavable-CARs was slightly lower than or equal to that of the normal CARs (FIG. 1-b). Because it is expected that the signals are turned ON/OFF due to the presence of the shedding structure, it was considered that the signal intensity was likely to be maintained from a long-term perspective. Accordingly, the coculture was conducted for 4 days, and then the reactivity was detected. As a result, it was demonstrated that significantly higher signal intensity was obtained from the Cleavable-CARs irrespective of the structure (FIG. 1-c). Since the long-term signals were enhanced, it is expected that the long-term therapeutic effects in vivo will be improved. In order to examine actual therapeutic effects, in-vivo examination was conducted using an experimental system in which CAR-introduced PBMCs were intravenously administered to a blood cancer model produced by intravenously administering NALM6 cells to NSG mice. As a result, it was found that, when the intracellular domain was 28bbz or 28z, the Cleavable-CARs significantly extended the survival time. In the case of bbz as well, the survival time tended to be extended (FIGS. 1-d, 1-e, 1-f). In addition, when a subcutaneous tumor models were examined using 28bbz CARs, it was demonstrated that the Cleavable-CARs exhibited higher effects than the normal CARs and extended the survival time (FIG. 1-g).

### Example 2: Examination of Mechanism by Which Shedding Leads to Potent CAR Effects

In order to investigate the mechanism by which the Cleavable-CARs could exhibit potent effects, first, the cytotoxicity of CD8⁺ T cells expressing CARs was detected, but there was no significant difference between the Normal-CARs and the Cleavable-CARs (FIGS. 2-a, 2-b). In recent years, it has been reported that the degree of trogocytosis by CARs results in a difference in the CAR reactivity. Therefore, various CARs were used to examine the extent that the CD19 molecules expressed by NALM6 cells during coculture with NALM6 cells were captured through trogocytosis. As a result, it was revealed that, irrespective of the types of intracellular domains in the CARs, a decrease in CD19 expression in the target cells was significantly suppressed when the Cleavable-CARs were used (FIG. 2-c). The difference in the trogocytosis level between the Normal-CARs and the Cleavable-CARs was the largest in the case of the 28bbz CARs. Accordingly, Tag proteins were added to the termini of the 28bbz CARs, and then the extracellular domains of the CARs reacted with the target were detected in images. As a result, it was observed that a portion of the Normal-CAR was taken in the target cell at an immunological synapse formation site, whereas the Cleavable-CAR attached to the target cell so as to cover the periphery thereof (FIGS. 2-d, 2-e). It was observed that, during the coculture with the target cells, the Normal-CARs pulled and bit off the cells, whereas it was observed that the Cleavable-CARs repeatedly came into contact with and separated from the cells, in other words, moved as if pecking the cells. It can be said that the results above demonstrate that the dynamic behavior of CAR-T cells changes depending on the presence or absence of the shedding structure in the receptor.

### Example 3: Examination of Trogocytosis Suppression Mechanism

In order to examine how the Cleavable-CARs suppress trogocytosis, an experiment on inhibition of a metalloprotease inducing shedding was conducted. First, an ADAM inhibitor, which induces cleavage of the extracellular domain, was added, and it was demonstrated that the suppression of trogocytosis was cancelled in a concentration-dependent manner even in the case of the Cleavable-CARs (FIG. 3-a). When a γ-secretase inhibitor was used to inhibit cleavage of the intracellular domain, trogocytosis was likely to occur as in the case above but to a smaller extent compared with the ADAM inhibitor. In the long-term stimulation experiment on NFAT-jurkat, it was observed that the addition of the ADAM inhibitor significantly reduces the signals, but no change was observed in the case of the γ-secretase inhibition (FIGS. 3-b, 3-c). Next, in order to examine if other shedding structures could cause similar trogocytosis suppression, a CAR in which the transmembrane domain of an amyloid precursor protein (APP) was used as the shedding structure was produced. As a result, in this case as well, the trogocytosis suppression could be observed. It was demonstrated that, when APP-TM into which mutation (Swe mutation) (known as the cause of the familial Alzheimer's disease), which enhances cleavage of the extracellular domain of APP, had been introduced was used, trogocytosis was further suppressed, which indicates that ease of cleavage of the extracellular domain of a CAR by a metalloprotease greatly defines the trogocytosis suppression level.

### Example 4: Examination of Relationship between Trogocytosis and Intracellular Signals

It was examined which signals defined the intensity of trogocytosis. First, in order to examine whether TCR signals via CD3ζ are required for trogocytosis in the Normal-CARs, trogocytosis was detected using constructs without CD3ζ. As a result, it was revealed that, although the trogocytosis level decreased, trogocytosis also occurred without the CD3ζ signals. It was demonstrated that, although there were no significant differences in the intensity of the NFAT signals, trogocytosis was more strongly induced by a construct having 28bb as the signal transduction domain than a construct having only the 4-1BB signal transduction domain or the CD28 signal transduction domain. Accordingly, based on the hypothesis that the size of the intracellular domain is more important than the signal intensity, the trogocytosis level was examined using a structure obtained by adding GFP, which is obviously considered not to be involved in signal transduction, to the N-terminus of a CAR. As a result, it was found that the GFP-added domain more strongly induced trogocytosis (FIG. 4-a). Next, in order to examine if the tyrosine kinase domain is necessary for trogocytosis, receptors in which portions of EGFP (SEQ ID NOS: 19 and 20) composed of various number of amino acids were connected to the downstream of the transmembrane domain were produced. As a result, it was demonstrated that trogocytosis occurred without the tyrosine kinase domain, and the trogocytosis level depended on the length of the intracellular domain (FIG. 5). Furthermore, when the non-signal transduction domain (EGFP267) and the tyrosine kinase domain (28bb), which have the same length, were compared, it is found that trogocytosis was more strongly induced in the case of the tyrosine kinase domain (FIG. 5). Also, it was revealed that trogocytosis could be more strongly suppressed by lining up signal domains having the same size near a portion of the membrane including the cleavage site as in 2828 (FIG. 6). In addition, when these CARs were examined using subcutaneous tumor models, it was demonstrated that the CAR into which the signal domains had been inserted in tandem extended the survival time of the mice (exhibited potent antitumor activity) (FIG. 7).

### Example 5: Examination of Relationship between Trogocytosis and Intracellular Domain

### Release

The transmembrane domain of the wild-type notch protein includes the S3 cleavage site and the S4 cleavage site that are cleavable by γ-secretase, and the intracellular domain of the notch protein is released into the cytoplasm by the γ-secretase cleaving the S3 and S4 cleavage sites. Accordingly, the trogocytosis suppressing effects were examined using a Cleavable-CAR (19Cleavable mutation 2828z) that cannot be cleaved by the γ-secretase due to mutation for deleting a portion of the S3 and S4 cleavage sites. After NALM6 cells and CAR-expressing Jurkat cells were cocultured, the NALM6 CD19 expression was measured. As a result, it was revealed that a decrease in the CD19 expression in the NALM6 cells was significantly suppressed (i.e., trogocytosis was further suppressed) in the case of the 19Cleavable mutation 2828z compared with the CAR (19Cleavable 2828z) having the transmembrane domain of the wild-type notch (FIG. 8). Accordingly, it is thought that the trogocytosis suppressing effects of the Cleavable-CAR are exhibited mainly due to cleavage of the extracellular domain by a sheddase, and release of the intracellular domain by the γ-secretase is not necessarily needed.

### Example 6: Examination of Trogocytosis by CAR Using Different Transmembrane Domain

The transmembrane (TM) site was changed to the TM of CD28, and then the trogocytosis suppressing effects were examined using the same trogocytosis detection system as in Example 5. Specifically, it was found that trogocytosis was further suppressed by linking the signal domains in tandem even in CARs in which the TM of human CD28 and 12 amino acids of the extracellular domain thereof as a hinge (FIG. 9). It is expected from these results that CD28 is also a shedding protein, and the cleavage site of the extracellular domain thereof is present within 12 amino acids from the TM.

### Example 7: Examination of Trogocytosis by CAR Using Different Ligand-Binding Site

Examination was conducted using a CAR having aTRBV12 ScFv or Her2 ScFV instead of CD19 ScFv. NFAT-Jurkat cells and various CAR-T cells into which aTRBV12ScFv had been incorporated were cocultured, and then the TCR signal intensity on the NFAT-jurkat was detected. It was confirmed that the signals tended to decrease when hCleavable-2828z CAR was used as the CAR (FIG. 10). The Her2 expression on the Her2-expressing tumor line skOV3 after overnight coculture of the skOV3 and various Her2-CARs was analyzed through FACS. It was demonstrated that, in the case of the Her2-CARs as well, trogocytosis was suppressed by the CARs having hCleavable, and in the case of using the 4-1BB signal domain as well, increasing the number of signal domains led to further suppression of trogocytosis when a transmembrane site having the shedding structure was used (FIG. 11).

### Example 8: Test on Impact of Length of Notch1 protein

The impact of shortening of the transmembrane domain having the shedding structure of human Notch1 on the reactivity of a CAR was tested. CARs (19hL31447 2828z, 19hL31448 2828z, 19hL31530 2828z, 19hL31560 2828z) with extracellular hinge domains having different length were produced and introduced into NFAT-Jurkat, and then the reactivity was checked by comparing the NFAT-Jurkat cocultured with a control or the NFAT-Jurkat cocultured with NALM6. The reactivity of the CAR having all the LNR, which has the amino acid residue at position 1447 as the first amino acid of the transmembrane domain, was as strong as that of hCleavable. As the LNR was shortened, the reactivity became weaker, but a significant reaction was observed compared with the control (FIG. 12). Accordingly, it is considered that changing the length of the Notch1 sequence used makes it possible to control the reactivity.

### Industrial Applicability

Using the present invention makes it possible to prevent trogocytosis in immune cells, allow a CAR agent such as Kymriah (Novartis Pharma K.K.) or YESCARTA (Gilead Sciences, Inc.) used as a gene therapy drug in clinical practice or a receptor such as a TCR to maintain the reactivity for a long period of time, and greatly enhance the effects of the existing T cell therapy. In addition, since there is no obvious harm in principle, early clinical application is possible. Furthermore, modifying the transmembrane domain and the intracellular domain of a receptor makes it possible to regulate trogocytosis. Advancing the research on the trogocytosis control technology of the present invention may lead to improvement of allogeneic transplantation technology, improvement of immune cell functions, and development of control technology for signal transduction via various cleavable proteins in the future.

The present application claims the benefit of priority based on Japanese Patent Application No. 2022-118674 filed in Japan (filing date: July 26, 2022), which is incorporated herein by reference in its entirety.

## Claims

1. An artificial receptor, comprising a ligand-binding site, a transmembrane domain having a shedding structure, and a signal transduction domain into an immune cell.

2. The artificial receptor according to claim 1, which is selected from the group consisting of a chimeric antigen receptor, a modified T cell receptor, a modified Fc receptor, and a modified tyrosine kinase receptor.

3. The artificial receptor according to claim 1 or 2, wherein the immune cell is selected from the group consisting of a T cell, a natural killer cell, and a macrophage.

4. The artificial receptor according to claim 3, wherein the T cell is a cytotoxic T cell.

5. The artificial receptor according to any one of claims 1 to 4, wherein the shedding structure is derived from a protein selected from the group consisting of a notch protein, an amyloid precursor protein, and CD28.

6. The artificial receptor according to any one of claims 1 to 5, wherein the signal transduction domain into an immune cell comprises a signal transduction domain derived from at least one type selected from the group consisting of a CD3ζ chain, CD28, and 4-1BB.

7. The artificial receptor according to any one of claims 1 to 6, wherein the transmembrane domain having a shedding structure comprises
(1) an amino acid sequence represented by SEQ ID NO: 26, or
(2) an amino acid sequence obtained by substituting, deleting, inserting, and/or adding one or several amino acids in the amino acid sequence of (1).

8. The artificial receptor according to any one of claims 1 to 7, having no γ-secretase cleavage site in the transmembrane domain.

9. The artificial receptor according to any one of claims 1 to 8, comprising two or more identical signal transduction domains.

10. A nucleic acid encoding the artificial receptor according to any one of claims 1 to 9.

11. An immune cell comprising the artificial receptor according to any one of claims 1 to 9 or the nucleic acid according to claim 10.

12. A medicine comprising the immune cell according to claim 11.

13. A method for designing a receptor with regulated trogocytosis effect or a nucleic acid encoding the receptor, comprising
(1) a step of selecting a receptor comprising a ligand-binding site, a transmembrane domain, and a signal transduction domain into an immune cell, and
(2A) a step of adjusting sensitivity of the transmembrane domain selected in the step (1) to shedding, and/or
(2B) a step of adding, inserting, and/or deleting one or more amino acid residues in the signal transduction domain of the receptor prepared in the step (1), and/or substituting one or more amino acid residues in the signal transduction domain.

14. A method for producing a receptor with regulated trogocytosis effect or a nucleic acid encoding the receptor, comprising
(1) a step of preparing a receptor that includes a ligand-binding site, a transmembrane domain, and a signal transduction domain into an immune cell, and
(2A) a step of adjusting sensitivity of the transmembrane domain prepared in the step (1) to shedding, and/or
(2B) a step of adding, inserting, and/or deleting one or more amino acid residues in the signal transduction domain of the receptor prepared in the step (1), and/or substituting one or more amino acid residues in the signal transduction domain.

15. The method according to claim 13 or 14, wherein the receptor selected or prepared in the step (1) is selected from the group consisting of a chimeric antigen receptor, a T cell receptor, an Fc receptor, and a tyrosine kinase receptor.
